# EUROPEAN PATENT APPLICATION

(11) **EP 4 215 199 A1**
(43) Date of publication of application: **26.07.2023**
(21) Application number: 21869457.8
(22) Date of filing: 17.09.2021
(51) Int. Cl.: A61K 31/7034, A61K 31/665, A61K 31/7032, A61K 31/7048, A61K 48/00, A61P 1/04, A61P 25/00, A61P 25/14, A61P 25/16, A61P 25/28, A61P 35/00

(54) **AUTOPHAGY ACTIVATOR**

(30) Priority: 17.09.2020 JP 2020156459
(71) Applicant: Resonac Corporation, Tokyo 105-8518 (JP)
(72) Inventor: NAKAGAMI Yuko, Tokyo 105-8518 (JP)
(74) Representative: Strehl Schübel-Hopf & Partner
(86) International application number: PCT/JP2021/034285
(87) International publication number: WO 2022/059775

(57) **Abstract**

There are provided an autophagy activator comprising methyl hesperidin as an active ingredient, and a composition for activating autophagy containing the autophagy activator and a pharmaceutically acceptable carrier.

## Description

### [Technical Field]

The present invention relates to an autophagy activator and a composition for activating autophagy.

Priority is claimed on Japanese Patent Application No. 2020-156459, filed September 17, 2020, the content of which is incorporated herein by reference.

### [Background Art]

Autophagy is a mechanism for regeneration of energy and removal of damaged substances by responding to extracellular or intracellular stresses and signals, such as starvation, growth factor deprivation, and pathogen infection, and by degrading aged or damaged intracellular substances and organelles, and is important for maintaining normal cellular homeostasis. Previous studies have reported that autophagy activity in cells decreases sharply as aging proceeds (Non-Patent Document 1). In addition, when autophagy is suppressed, aging mitochondria and misfolded proteins accumulate excessively in cells, and oxidative stress increases in cells, which induces cell death, and as a result, cellular senescence occurs.

Therefore, by activating autophagy that degrades aging substances in cells and organelles and recycles degradation products, cellular homeostasis can be enhanced by rapidly removing intracellular waste.

On the other hand, as an effect of aging on autophagy, it is known that the expression levels of ATG5, ATG7, and Beclin 1 genes decrease with aging in the human brain. In addition, in neurodegenerative diseases such as Alzheimer's disease, decreased autophagy activity is believed to be observed. Therefore, activation of autophagy is known to contribute to the treatment and prevention of neurodegenerative diseases such as Alzheimer's disease, Huntington's disease, and Parkinson's disease (Non-Patent Documents 2 and 3). Especially in Alzheimer's disease, since the function of autophagy is inhibited, aggregated protein called amyloid β accumulates in the living body, and this is said to be involved in the development of the disease (Non-Patent Document 4). In addition, mutations in autophagy-related genes and genes involved in selective autophagy are said to affect static encephalopathy of children with neurodegeneration in adulthood (SENDA) disease, which is a neurodegenerative disease accompanied by iron deposition in the substantia nigra and globus pallidus and cerebral atrophy, Crohn's disease, which is an inflammatory bowel disease that causes severe inflammation or ulcers in the digestive tract, and cancer (Non-Patent Document 5).

The autophagy process has been studied in both yeast and mammals and utilizes up to 36 proteins. In particular, the process from autophagosome formation to differentiation of the contents thereof is controlled by the Atg protein encoded by the autophagy-related gene (ATG), classification into six groups including the Atg12-Atg5 binding system and the LC3-phosphatidyl ethanolamine (PE) binding system can be performed, and each works step by step in each process.

Autophagy, on the other hand, is suppressed to a low level in a steady state, but is activated under stress such as starvation. The mammalian target of rapamycin (mTOR) works as a major suppressor of autophagy in yeast and mammals, but it is known that mTOR is inactivated and autophagy is induced under starvation conditions (Non-Patent Document 6).

As autophagy activators, compounds that increase LC3-related factors, which are markers of autophagy activity and activate autophagy, and compounds that promote autophagic flux (including fusion of autophagosomes to lysosomes) have been reported (Patent Documents 1 to 4).

In addition, hesperidin, which is a type of polyphenol extracted from citrus fruits, is known to have an effect of activating autophagy (Non-Patent Document 7).

### [Citation List]

### [Patent Documents]

[Patent Document 1]
   PCT International Publication No. 2018/173653
[Patent Document 2]
   Japanese Unexamined Patent Application, First Publication No. 2018-80204
[Patent Document 3]
   PCT Japanese Translation Patent Publication No. 2018-510902
[Patent Document 4]
   PCT Japanese Translation Patent Publication No. 2019-529514

### [Non-Patent Document]

[Non-Patent Document 1] Yogendra S. Rajawat et al., Aging: Central role for autophagy and the lysosomal degradative system. Aging Research Reviews 8 (2009) 199-213.
[Non-Patent Document 2] Aaron Barnett et al., Autophagy in Aging and Alzheimer's Disease: Pathologic or Protective?. J Alzheimers Dis. 2011; 25(3): 385-394.
[Non-Patent Document 3] Marta M. Lipinski et al., Genome-wide analysis reveals mechanisms modulating autophagy in normal brain aging and in Alzheimer's disease. Proc Natl Acad Sci USA. 2010, 107, 14164-14169.
[Non-Patent Document 4] Xiangqing Li et al., Cubeben induces autophagy via PI3K-AKT-mTOR pathway to protect primary neurons against amyloid beta in Alzheimer's disease. Cytotechnology (2019) 71: 679-686.
[Non-Patent Document 5] Kageyama et al., "Autophagy and Disease", Interdisciplinary Review, 2014, 3, e006
[Non-Patent Document 6] Inomata et al., "Relationship between autophagy and aging," Journal of Dental Science, 2018, Vol. 45, No. 1, 1-7
[Non-Patent Document 7] Gowrikumar Saiprasad et al., Hesperidin induces apoptosis and triggers autophagic markers through inhibition of Aurora-A mediated phosphoinositide-3-kinase/Akt/mammalian target of rapamycin and glycogen synthase kinase-3 beta signalling cascades in experimental colon carcinogenesis. European Journal of Cancer (2014) 50, 2489-2507.

### [Summary of Invention]

### [Technical Problem]

As described above, it is known that the autophagy function deteriorates in various diseases such as Alzheimer's disease, and drugs capable of effectively activating autophagy are desired. However, it can be said that effects of conventionally known drugs are still insufficient.

Accordingly, an object of the present invention is to provide an autophagy activator and a composition for activating autophagy containing the autophagy activator capable of effectively activating autophagy.

### [Solution to Problem]

The present invention includes the following aspects.
[1] An autophagy activator containing methyl hesperidin as an active ingredient.
[2] The autophagy activator according to [1], in which the methyl hesperidin is one or more selected from the group consisting of chalcone methyl hesperidin represented by the following general Formula (1) and flavanone methyl hesperidin represented by the following general Formula (2). 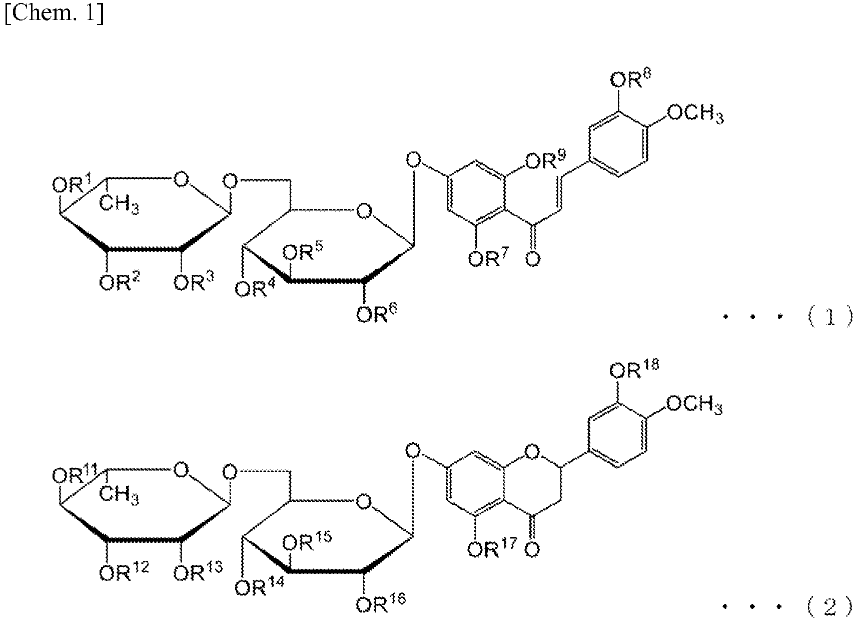
[In Formula (1), R¹ to R⁹ are each independently a methyl group or a hydrogen atom, provided that at least one of R¹ to R⁹ is a methyl group.
In Formula (2), R¹¹ to R¹⁸ are each independently a methyl group or a hydrogen atom, provided that at least one of R¹¹ to R¹⁸ is a methyl group.]

[3] The autophagy activator according to [2], in which the methyl hesperidin is one or more selected from the group consisting of chalcone methyl hesperidin represented by the following general Formula (3) and flavanone methyl hesperidin represented by the following general Formula (4). 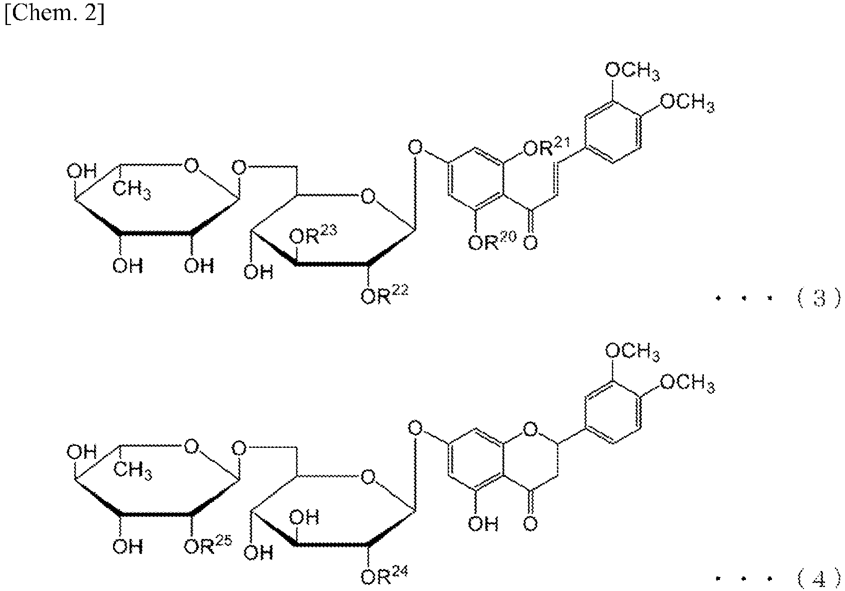
[In Formula (3), R²⁰ to R²³ are each independently a methyl group or a hydrogen atom.
In Formula (4), R²⁴ and R²⁵ are each independently a methyl group or a hydrogen atom.]

[4] The autophagy activator according to [3], in which the chalcone methyl hesperidin represented by the general Formula (3) is one or more selected from the group consisting of chalcone-1 to chalcone-3 having a combination of R²⁰ to R²³ shown in Table 1 below.

**[Table 1]**

| | R²⁰ | R²¹ | R²² | R²³ |
|---|---|---|---|---|
| Chalcone-1 | CH₃ | CH₃ | CH₃ | CH₃ |
| Chalcone-2 | H | CH₃ | CH₃ | H |
| Chalcone-3 | H | CH₃ | H | H |

[5] The autophagy activator according to [3] or [4], in which the flavanone methyl hesperidin represented by the general Formula (4) is one or more selected from the group consisting of flavanone-1 to flavanone-4 having a combination of R²⁴ and R²⁵ shown in Table 2 below.

**[Table 2]**

| | R²⁴ | R²⁵ |
|---|---|---|
| Flavanone-1 | CH₃ | CH₃ |
| Flavanone-2 | CH₃ | H |
| Flavanone-3 | H | H |
| Flavanone-4 | H | CH₃ |

[6] The autophagy activator according to any one of [1] to [5], in which LC3 gene expression is promoted.
[7] The autophagy activator according to any one of [1] to [6], in which ATG5 gene expression is promoted.
[8] The autophagy activator according to any one of [1] to [7], in which ATG7 gene expression is promoted.
[9] The autophagy activator according to any one of [1] to [8], in which mTOR gene expression is suppressed.
[10] The autophagy activator according to any one of [1] to [9], which is used for prevention or treatment of Alzheimer's disease.
[11] A composition for activating autophagy, containing the autophagy activator according to any one of [1] to [10] and a pharmaceutically acceptable carrier.
[12] The composition for activating autophagy according to [11], in which the total amount of methyl hesperidin is 0.01 to 2% by mass relative to the total amount of the composition for activating autophagy.
[13] The composition for activating autophagy according to [11] or [12], further containing at least one vitamin derivative or a salt thereof selected from the group consisting of vitamin C derivatives and vitamin E derivatives.
[14] The composition for activating autophagy according to [13], in which the vitamin derivative or a salt thereof is at least one selected from the group consisting of ascorbyl phosphate, fatty acid esters of ascorbyl phosphate, tocopherol phosphate, and salts thereof.
[15] The composition for activating autophagy according to any one of [11] to [14], further containing an inositol derivative in which a saccharide is bound to inositol.
[16] The composition for activating autophagy according to [15], in which the saccharide is glucose or an oligosaccharide containing glucose as a constituent unit.

### [Advantageous Effects of Invention]

According to the present invention, it is possible to provide an autophagy activator and a composition for activating autophagy containing the autophagy activator capable of effectively activating autophagy.

### [Description of Embodiments]

### (Autophagy activator)

In one embodiment, the present invention provides an autophagy activator containing methyl hesperidin as an active ingredient.

Here, "autophagy" is a mechanism that regenerates energy and removes damaged substances by degrading aged or damaged intracellular substances and organelles.

The autophagy activator of the present embodiment can promote the expression of the LC3 gene, which is an autophagy marker, and the ATG5 and ATG7 genes contained in autophagosomes, and can activate autophagy. In addition, autophagy can be activated by suppressing the expression of the mTOR gene, which acts as a suppressor of autophagy.

### <Methyl hesperidin>

The autophagy activator of the present embodiment is not particularly limited as long as the autophagy activator contains methyl hesperidin as an active ingredient. Methyl hesperidin used in the autophagy activator of the present embodiment is preferably hesperidin methylated and solubilized in water.

It is known that methyl hesperidin mainly is a chalcone type compound (chalcone methyl hesperidin) represented by the following general Formula (1) and a flavanone type compound (flavanone methyl hesperidin) represented by the following general Formula (2).

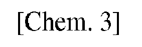

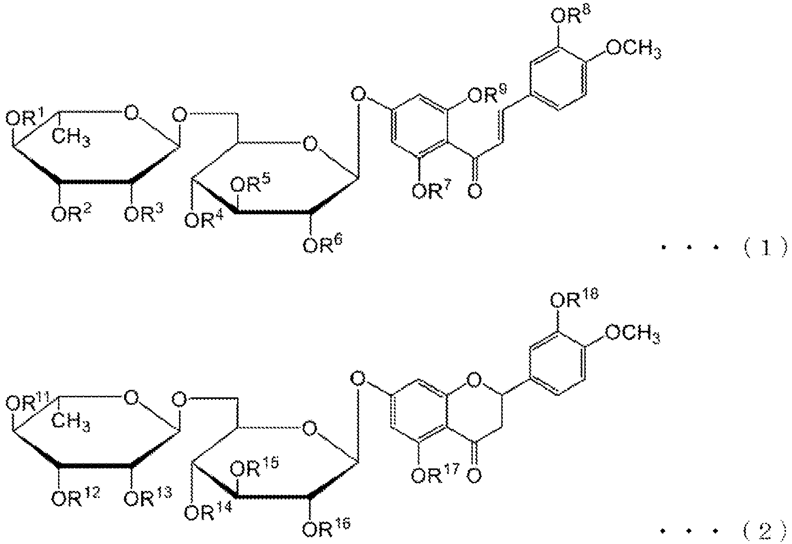

[In Formula (1), R¹ and R⁹ are each independently a methyl group or a hydrogen atom, provided that at least one of R¹ to R⁹ is a methyl group.

In Formula (2), R¹¹ to R¹⁸ are each independently a methyl group or a hydrogen atom, provided that at least one of R¹¹ to R¹⁸ is a methyl group.]

[3] Preferably, the methyl hesperidin used in the autophagy activator of the present embodiment is one or more selected from the group consisting of chalcone methyl hesperidin represented by the general Formula (1) and flavanone methyl hesperidin represented by the general Formula (2).

In the general Formula (1), R¹ to R⁹ are each independently a methyl group or a hydrogen atom, and at least one of R¹ to R⁹ is a methyl group. Among R¹ to R⁹, preferably, any one to six are methyl groups, and more preferably, any two to five are methyl groups.

In the general Formula (2), R¹¹ to R¹⁸ are each independently a methyl group or a hydrogen atom, and at least one of R¹¹ to R¹⁸ is a methyl group. Among R¹¹ to R¹⁸, preferably, any one to four are methyl groups, and more preferably, any one to three are methyl groups.

Among the compounds represented by the general Formula (1), a compound represented by the following general Formula (3) is preferable as the chalcone methyl hesperidin. Among the compounds represented by the general Formula (2), a compound represented by the following general Formula (4) is preferable as the flavanone methyl hesperidin.

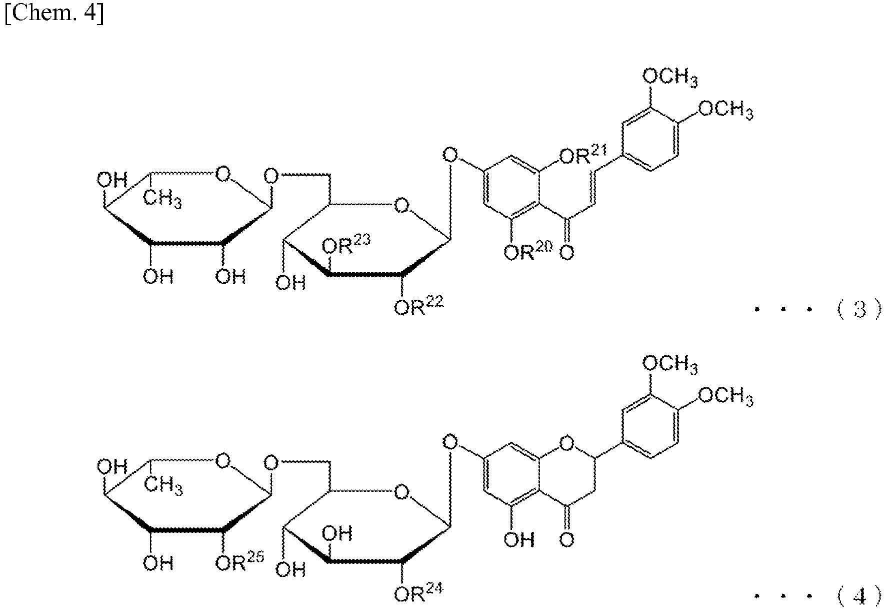

[In Formula (3), R²⁰ to R²³ are each independently a methyl group or a hydrogen atom.

In Formula (4), R²⁴ and R²⁵ are each independently a methyl group or a hydrogen atom.]

In the general Formula (3), R²⁰ to R²³ are each independently a methyl group or a hydrogen atom. The chalcone methyl hesperidin represented by the general Formula (3) is preferably one or more selected from the group consisting of chalcone-1 to chalcone-3 having combinations of R²⁰ to R²³ shown in Table 3 below.

**[Table 3]**

| | R²⁰ | R²¹ | R²² | R²³ |
|---|---|---|---|---|
| Chalcone-1 | CH₃ | CH₃ | CH₃ | CH₃ |
| Chalcone-2 | H | CH₃ | CH₃ | H |
| Chalcone-3 | H | CH₃ | H | H |

In the general Formula (4), R²⁴ and R²⁵ are each independently a methyl group or a hydrogen atom. The flavanone methyl hesperidin represented by the general Formula (4) is preferably one or more selected from the group consisting of flavanone-1 to flavanone-4 having combinations of R²⁴ and R²⁵ shown in Table 4 below.

**[Table 4]**

| | R²⁴ | R²⁵ |
|---|---|---|
| Flavanone-1 | CH₃ | CH₃ |
| Flavanone-2 | CH₃ | H |
| Flavanone-3 | H | H |
| Flavanone-4 | H | CH₃ |

One type of methyl hesperidin used in the autophagy activator of the present embodiment may be used alone or a mixture of two or more types thereof may be used. Methyl hesperidin may include both a chalcone methyl hesperidin represented by the general Formula (1) or a chalcone methyl hesperidin represented by the general Formula (3), and a flavanone methyl hesperidin represented by the general Formula (2) or a flavanone methyl hesperidin represented by the general Formula (4), or may contain only one of them. The methyl hesperidin may include the chalcone methyl hesperidin represented by the general Formula (3) and the flavanone methyl hesperidin represented by the general Formula (4). In addition, the methyl hesperidin may include any one or more of the above-mentioned chalcone-1 to chalcone-3, and may include any one or more of the above-mentioned flavanone-1 to flavanone-4.

Further, the autophagy activator of the present embodiment may include a mixture of chalcone-1 to chalcone-3 and flavanone-1 to flavanone-3 as methyl hesperidin.

Methyl hesperidin can be produced by a known method. Methyl hesperidin can be produced by, for example, dissolving hesperidin produced from citrus peel in an aqueous sodium hydroxide solution, allowing the alkali solution to act with a corresponding amount of dimethylsulfuric acid, neutralizing the reaction solution with sulfuric acid, extracting with n-butyl alcohol, distilling off the solvent, and recrystallizing with isopropyl alcohol (Sakieki, Nippon Kagaku Zassi, (1958) Vol. 79, pp. 733-736; Japanese Patent No. 6312333). The method for producing methyl hesperidin is not limited to the above method.

Methyl hesperidin can also be used by purchasing a commercial product (for example, those distributed as pharmaceutical additives, food additives, and cosmetic ingredients, or "Methyl Hesperidin" (Showa Denko K.K.), "Methyl Hesperidin" (Tokyo Chemical Industry Co., Ltd.), "Hesperidin Methyl Chalcone" (Sigma-Aldrich) or the like).

The autophagy activator of the present embodiment can be used by being administered to a patient for the purpose of treating neurodegenerative diseases such as Alzheimer's disease, Huntington's disease, and Parkinson's disease. In addition, the autophagy activator of the present embodiment can also be used by blending the autophagy activator into pharmaceuticals and cosmetics for the purpose of activating autophagy. Moreover, the autophagy activator may be used by blending the autophagy activator into the composition for activating autophagy described below.

The autophagy activator of the present embodiment can effectively activate autophagy by promoting LC3 gene expression.

The autophagy activator of the present embodiment can effectively activate autophagy by promoting ATG5 gene expression.

The autophagy activator of the present embodiment can effectively activate autophagy by promoting ATG7 gene expression.

The autophagy activator of the present embodiment can effectively activate autophagy by suppressing mTOR gene expression.

Since the autophagy activator of the present embodiment can effectively activate autophagy, the autophagy activator can be used for prevention or treatment of Alzheimer's disease.

Amyloid β is known to cause a decrease in autophagy in nerve cells. In addition, amyloid β is known to induce a decrease in autophagy and thereby cell death called apoptosis of nerve cells.

The autophagy activator of the present embodiment can promote LC3 gene expression in the presence of amyloid β.

The autophagy activator of the present embodiment can promote ATG5 gene expression in the presence of amyloid β.

The autophagy activator of the present embodiment can promote ATG7 gene expression in the presence of amyloid β.

The autophagy activator of the present embodiment can suppress apoptosis in the presence of amyloid β.

The autophagy activator of the present embodiment can promote expression of at least one gene selected from the group consisting of the LC3 gene, ATG5 gene, and ATG7 gene in the presence of amyloid β, particularly in nerve cells. In addition, the autophagy activator of the present embodiment can suppress apoptosis in the presence of amyloid β, particularly in nerve cells.

Promoting LC3 gene expression in the presence of amyloid β means that administration of the autophagy activator of the present embodiment in the presence of amyloid β results in an increase in expression level of the LC3 gene compared to the case where the autophagy activator is not administered. The same is also applied for the ATG5 gene and the ATG7 gene.

Suppressing apoptosis in the presence of amyloid β means that administration of the autophagy activator of the present embodiment in the presence of amyloid β results in suppressing apoptosis in expression compared to the case where the autophagy activator is not administered.

LC3 (microtubule associated protein 1 light chain 3 alpha: NCBI Gene ID: 84557) to which phosphatidylethanolamine has been added upstream of autophagy signal transduction is converted into LC3-II that is attracted to the autophagosome membrane, and binds to the autophagosome membrane. LC3 is used as a marker for autophagosomes. Examples of nucleotide sequences of the human LC3 gene include NM_032514.4 and NM_181509.3 registered in the NCBI Reference Sequence database.

ATG5 (autophagy related 5: NCBI Gene ID: 9474) binds to ATG12 and functions as an E1-like activating enzyme in a ubiquitin-like conjugation system. Examples of nucleotide sequences of the human ATG5 gene include NM_001286106.1, NM_001286107.1, NM_001286108.1, NM_001286111.1, and NM_004849.4 registered in the NCBI Reference Sequence database.

ATG7 (autophagy related 7: NCBI Gene ID: 10533) functions as an E1-like activating enzyme that activates LC3 and ATG12 in an ATP-dependent manner. Examples of nucleotide sequences of the human ATG7 gene include NM_001136031.3, NM_001144912.2, NM_001349232.2, NM_001349233.2, and NM_001349234.2 registered in the NCBI Reference Sequence database.

mTOR (mechanistic target of rapamycin kinase: NCBI Gene ID: 2475) is one type of phosphatidylinositol kinase-related kinase, and mediates cellular responses to stresses such as DNA damage and nutrient deprivation. mTOR functions as a suppressor of autophagy. Examples of nucleotide sequences of the human mTOR gene include NM_004958.4 and the like registered in the NCBI Reference Sequence database.

The autophagy activator of the present embodiment may be used by being administered to patients at high risk of developing neurodegenerative diseases such as Alzheimer's disease, Huntington's disease, and Parkinson's disease and prevent neurodegenerative diseases such as Alzheimer's disease, Huntington's disease, and Parkinson's disease. In addition, the autophagy activator of the present embodiment may be used by being administered to patients who have developed neurodegenerative diseases such as Alzheimer's disease, Huntington's disease, or Parkinson's disease, and suppress the progression or aggravation of the neurodegenerative disease.

The autophagy activator of the present embodiment can be administered to patients in the same manner as the composition for activating autophagy described below, may be administered orally, may be administered parenterally, may be administered intravenously, intraarterially, intramuscularly, intradermally, subcutaneously, intraperitoneally, or the like, may be administered intrarectally as a suppository; or may be administered to the skin as an external skin preparation.

### (Composition for activating autophagy)

The composition for activating autophagy of the present embodiment contains the above-described autophagy activator containing methyl hesperidin and a pharmaceutically acceptable carrier.

The composition for activating autophagy of the present embodiment can be produced according to a conventional method (for example, the method described in the Japanese Pharmacopoeia) by mixing and formulating the above-described autophagy activator, a pharmaceutically acceptable carrier, and optionally other ingredients.

As used herein, the term "pharmaceutically acceptable carrier" means a carrier that does not inhibit the physiological activity of the active ingredient and does not exhibit substantial toxicity to the administration target.

The term "not exhibit substantial toxicity" means that the ingredient does not show toxicity to the administration target at the administration dosage normally used.

Pharmaceutically acceptable carriers are not particularly limited, and examples thereof include excipients, binders, disintegrating agents, lubricants, stabilizers, diluents, solvents for injection, moisturizers, texture improvers, surfactants, polymerizing/thickening/gelling agents, solvents, propellants, antioxidants, reducing agents, oxidizing agents, chelating agents, acids, alkalis, powders, inorganic salts, water, metal-containing compounds, unsaturated monomers, polyhydric alcohols, polymer additives, wetting agents, thickeners, tackifiers, oily raw materials, liquid matrices, fat-soluble substances, and polymer carboxylates.

Specific examples of these ingredients include those described in International Publication No. 2016/076310. Furthermore, specific examples of the polymerizing/thickening/gelling agent include methacryloyloxyethyl phosphorylcholine, butyl methacrylate, and polymers thereof.

The pharmaceutically acceptable carriers in the composition for activating autophagy of the present embodiment may be used alone or in combination of two or more.

In addition, other ingredients are not particularly limited, and examples thereof include preservatives, antibacterial agents, ultraviolet absorbers, whitening agents, vitamins other than methyl hesperidin and the derivatives thereof, antiphlogistic agents, anti-inflammatory agents, hair growth agents, blood circulation promoters, stimulants, hormones, anti-wrinkle agents, anti-aging agents, tightening agents, cooling agents, warming agents, wound healing accelerators, irritation mitigation agents, analgesics, cell activators, plant/animal/microorganism extracts, seed oil, antipruritic agents, exfoliating/dissolving agents, antiperspirants, algefacients, astringents, enzymes, nucleic acids, fragrances, pigments, coloring agents, dyes, pigments, anti-inflammatory analgesics, antifungal agents, antihistamines, hypnotic sedatives, tranquilizers, antihypertensive agents, antihypertensive diuretics, antibiotics, anesthetics, antibacterial substances, antiepileptic agents, coronary vasodilators, herbal medicines, antipruritic agents, keratin softening exfoliants, UV blockers, bactericides, antioxidants, pH adjusters, additives, and metal soaps. Specific examples of these ingredients include those described in International Publication No. 2016/076310. Furthermore, specific examples of plant/animal/microorganism extracts include Lapsana communis flower/leaf/stem, and tea leaves. Specific examples of seed oils include moringa seed oil. Specific examples of fragrances include perillaldehyde.

The other ingredients may be used alone, or in combination of two or more.

The composition for activating autophagy of the present embodiment can contain a therapeutically effective amount of the autophagy activator. "Therapeutically effective amount" means an amount of drug effective to treat or prevent disease in a patient. The therapeutically effective amount may vary depending on the disease state, age, sex, body weight, and the like of the administration target.

In the composition for activating autophagy of the present embodiment, therapeutically effective amount of the autophagy activator may be an amount that allows methyl hesperidin to activate autophagy. In addition, the therapeutically effective amount of the autophagy activator can be an amount that allows methyl hesperidin to promote the expression of at least one gene selected from the group consisting of the LC3 gene, ATG5 gene, and ATG7 gene. Further, the therapeutically effective amount of the autophagy activator may be an amount that allows methyl hesperidin to suppress mTOR gene expression. In addition, the therapeutically effective amount of the autophagy activator may be an amount that allows methyl hesperidin to suppress apoptosis in the presence of amyloid β.

The therapeutically effective amount of the autophagy activator (total amount of methyl hesperidin) in the composition for activating autophagy of the present embodiment may be, for example, 0.01 to 2% by mass, may be, for example, 0.05 to 1.5% by mass, and may be, for example, 0.1 to 1.0% by mass relative to the total amount of the composition for activating autophagy.

In addition, the total amount of methyl hesperidin means the amount of the compound when one type of methyl hesperidin is used alone, and the total amount of these compounds when two or more types of methyl hesperidin are used in combination.

The composition for activating autophagy of the present embodiment may contain other autophagy activating components in addition to the autophagy activator. Examples of the other autophagy activating components include at least one vitamin derivative or a salt thereof selected from the group consisting of vitamin C derivatives and vitamin E derivatives, and inositol derivatives in which a saccharide is bound to inositol.

<Vitamin C derivative or salt thereof>

The composition for activating autophagy of the present embodiment preferably contains a vitamin C derivative or a salt thereof in addition to the autophagy activator. By containing a vitamin C derivative or a salt thereof, the activation action of autophagy is further improved.

Examples of vitamin C derivatives include ascorbic acid derivatives obtained by derivatizing at least one hydroxyl group of ascorbic acid.

More specifically, examples of the ascorbic acid derivative include ascorbyl phosphate obtained by phosphorylating any of the hydroxyl groups of ascorbic acid (also referred to as ascorbic acid phosphate); fatty acid esters of ascorbyl phosphate obtained by phosphorylating any of the hydroxyl groups of ascorbic acid, and esterifying the other hydroxyl groups with fatty acids; ethyl ascorbic acid obtained by ethoxylating any of the hydroxyl groups of ascorbic acid; ascorbic acid glucoside obtained by glucosidating any of the hydroxyl groups of ascorbic acid; acylated ascorbic acid obtained by acylating any of the hydroxyl groups of ascorbic acid; acylated ascorbyl phosphate obtained by acylating any of the hydroxyl groups of ascorbic acid and phosphorylating the other hydroxyl groups; glyceryl ascorbic acid obtained by substituting any of hydroxyl groups of ascorbic acid with glycerin; and phosphoric acid diester of ascorbic acid and tocopherol obtained by binding each of ascorbic acid and tocopherol by ester bond via phosphoric acid (specifically, d1-α-tocopherol 2-L-ascorbic acid phosphate diester, and the like).

Examples of salts of ascorbic acid derivatives include salts of ascorbic acid derivatives and inorganic bases, and salts of ascorbic acid derivatives and organic bases.

Examples of salts with inorganic bases include alkali metal salts such as sodium salts and potassium salts; alkaline earth metal salts such as calcium salts and magnesium salts; aluminum salts; ammonium salts; and zinc salts.

Examples of salts with organic bases include alkylammonium salts and salts with basic amino acids.

Among the above, as ascorbic acid derivatives or salts thereof, (i) ascorbyl phosphate or salts thereof, (ii) fatty acid esters of ascorbyl phosphate or salts thereof, (iii) ethyl ascorbic acid or salts thereof, and (iv) ascorbic acid glucoside or salts thereof are preferable, and (i) ascorbyl phosphate or salts thereof and (ii) fatty acid esters of ascorbyl phosphate or salts thereof are more preferable.

### <<(i) Ascorbyl phosphate or salts thereof>>

### · Ascorbyl Phosphate

Ascorbyl phosphate is a compound in which a phosphate group is introduced to at least one hydroxyl group of ascorbic acid.

Suitable examples of ascorbyl phosphate include a compound represented by the following chemical Formula (5).

The compound represented by the following chemical Formula (5) is ascorbic acid-2-phosphate obtained by protecting the hydroxyl group at the second position of ascorbic acid with phosphate.

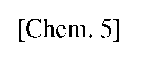

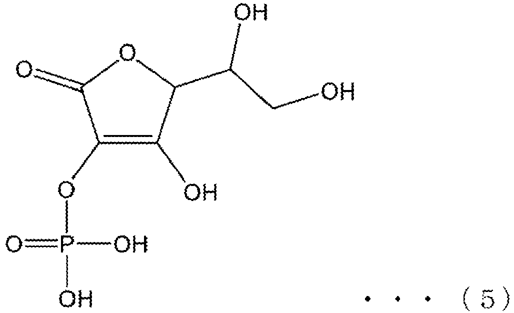

Ascorbyl phosphate exists as D- and L-stereoisomers, as well as the racemic DL-form. Ascorbyl phosphate in the present embodiment may be any of these stereoisomers, but from the viewpoint of easy availability, the L-form is preferable, and specifically, L-ascorbic acid-2-phosphate esters are preferable.

### · Salts of ascorbyl phosphate

Examples of salts of ascorbyl phosphate include salts of ascorbyl phosphate and inorganic bases, and salts of ascorbyl phosphate and organic bases.

Examples of salts with inorganic bases include alkali metal salts such as sodium salts and potassium salts; alkaline earth metal salts such as calcium salts and magnesium salts; aluminum salts; ammonium salts; and zinc salts.

Examples of salts with organic bases include alkylammonium salts and salts with basic amino acids.

Among the above, as the salts of ascorbyl phosphate, alkali metal salts or alkaline earth metal salts are preferable, sodium salts or magnesium salts are more preferable, and magnesium salts are still more preferable.

A magnesium salt of ascorbyl phosphate is preferable from the viewpoint of high stability and resistance to coloration.

Among the above, as the ascorbyl phosphate or salts thereof, from the viewpoint of improving stability, a salt of ascorbyl phosphate is preferable, an alkali metal salt of the compound represented by the above chemical Formula (5) or an alkaline earth metal salt of the compound represented by (5) is more preferable, and a sodium salt of the compound represented by the chemical Formula (5) or a magnesium salt of the compound represented by the chemical Formula (5) is still more preferable.

Specifically, as the magnesium salt of the compound represented by the above chemical Formula (5), the magnesium salt of L-ascorbic acid-2-phosphate is particularly preferable.

Specifically, as the sodium salt of the compound represented by the above chemical Formula (5), the sodium salt of L-ascorbic acid-2-phosphate is particularly preferable.

In the composition for activating autophagy of the present embodiment, ascorbyl phosphate or salts thereof may be used alone or in combination of two or more.

When the composition for activating autophagy of the present embodiment contains ascorbyl phosphate or a salt thereof, the amount thereof is preferably 0.1 to 15% by mass, more preferably 0.5 to 10% by mass, and still more preferably 1 to 5% by mass relative to the total amount of the composition for activating autophagy.

Ascorbyl phosphate or salts thereof can be produced by known production methods such as those described in Japanese Unexamined Patent Publication No. 2-279690 and Japanese Unexamined Patent Publication No. 6-345786.

For example, as a specific method for producing ascorbyl phosphate, ascorbyl phosphate can be obtained by reacting ascorbic acid with phosphorus oxychloride or the like for phosphorylation.

Further, as a specific method for producing a salt of ascorbyl phosphate, a salt of ascorbyl phosphate can be obtained by neutralizing the ascorbyl phosphate solution with a metal oxide such as magnesium oxide or a metal hydroxide such as sodium hydroxide.

Examples of commercially available ascorbyl phosphate salts include ascorbic acid PS (compound name: sodium salt of L-ascorbic acid-2-phosphate ester (also referred to as sodium L-ascorbyl-2-phosphate), display name: Sodium Ascorbyl Phosphate) manufactured by Showa Denko K.K., and ascorbic acid PM (compound name: magnesium salt of L-ascorbic acid-2-phosphate ester (also referred to as magnesium L-ascorbyl-2-phosphate), display name; Magnesium Ascorbyl Phosphate) manufactured by Showa Denko K.K.

### <<(ii) Fatty acid esters of ascorbyl phosphate or salts thereof>>

### · Fatty acid esters of ascorbyl phosphate

The fatty acid ester of ascorbyl phosphate is a compound in which a fatty acid is ester-bound to at least one hydroxyl group of ascorbyl phosphate. As the fatty acid, a linear or branched fatty acid having 6 to 22 carbon atoms (that is, a fatty acid having a linear or branched alkyl group bound to the carboxy group having 5 to 21 carbon atoms) is preferable, a linear or branched fatty acid having 10 to 20 carbon atoms is more preferable, and a linear or branched fatty acid having 12 to 18 carbon atoms is still more preferable.

Examples of fatty acid esters of ascorbyl phosphate include a compound represented by the following general Formula (6). The compound represented by the following general Formula (6) is an ascorbic acid-2-phosphoric acid-6-fatty acid in which phosphoric acid is ester-bound to the hydroxyl group at the second position of ascorbic acid and fatty acid is ester-bound to the hydroxyl group at the sixth position. 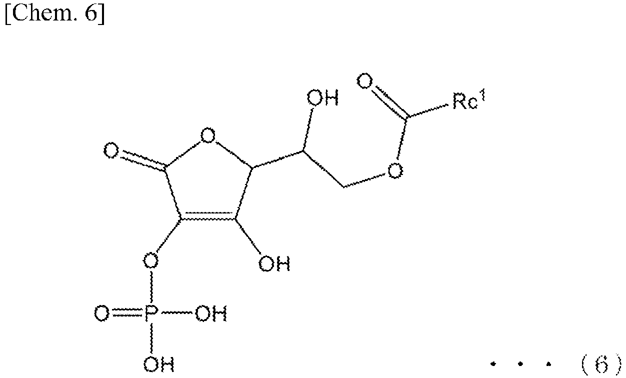 [In the formula, Rc¹ is a linear or branched alkyl group having 5 to 21 carbon atoms]

In the general Formula (6), Rc¹ is a linear or branched alkyl group having 5 to 21 carbon atoms. Specifically, examples thereof include a linear or branched pentyl group, a linear or branched hexyl group, a linear or branched heptyl group, a linear or branched octyl group, a linear or branched nonyl group, a linear or branched decyl group, a linear or branched undecyl group, a linear or branched dodecyl group, a linear or branched tridecyl group, a linear or branched tetradecyl group, a linear or branched pentadecyl group, a linear or branched hexadecyl group, a linear or branched heptadecyl group, a linear or branched octadecyl group, a linear or branched nonadecyl group, a linear or branched icosyl group, and a linear or branched henicosyl group.

In the above general Formula (6), Rc¹ is preferably a linear or branched alkyl group having 9 to 19 carbon atoms, more preferably a linear or branched alkyl group having 11 to 17 carbon atoms, still more preferably a linear or branched alkyl group having 13 to 15 carbon atoms, and from the viewpoint of availability of raw materials, particularly preferably a linear alkyl group having 15 carbon atoms (linear pentadecyl group).

That is, 6-O-palmitoyl ascorbic acid-2-phosphate (also referred to as ascorbic acid-2-phosphate-6-palmitic acid) is particularly preferable as the compound represented by the general Formula (6).

The fatty acid esters of ascorbyl phosphate exist as D- and L-stereoisomers, as well as the racemic DL-form. The fatty acid ester of ascorbyl phosphate in the present embodiment may be any of these stereoisomers, but from the viewpoint of easy availability, the L-form is preferable, and specifically, the fatty acid esters of L-ascorbic acid-2-phosphate ester are preferable.

### · Salts of fatty acid esters of ascorbyl phosphate

Examples of salts of fatty acid esters of ascorbyl phosphate include salts of fatty acid esters of ascorbyl phosphate and inorganic bases, and salts of fatty acid esters of ascorbyl phosphate and organic bases.

Examples of salts with inorganic bases include alkali metal salts such as sodium salts and potassium salts; alkaline earth metal salts such as calcium salts and magnesium salts; aluminum salts; ammonium salts; and zinc salts.

Examples of salts with organic bases include alkylammonium salts and salts with basic amino acids.

Among the above, as the salts of fatty acid esters of ascorbyl phosphate, alkali metal salts or alkaline earth metal salts are preferable, sodium salts or magnesium salts are more preferable, and sodium salts are still more preferable.

Sodium salts of fatty acid esters of ascorbyl phosphate are preferable from the viewpoint of stability and ease of incorporation into formulations.

Among the above, as the fatty acid ester of the ascorbyl phosphate or a salt thereof, from the viewpoint of stability and ease of incorporation into formulations, a salt of fatty acid ester of ascorbyl phosphate is preferable, an alkali metal salt of the compound represented by the above general Formula (6) or an alkaline earth metal salt of the compound represented by the above general Formula (6) is more preferable, a sodium salt of the compound represented by the general Formula (6) or a magnesium salt of the compound represented by the general Formula (6) is still more preferable, and a sodium salt of the compound represented by the general Formula (6), specifically a sodium salt of L-ascorbic acid-2-phosphoric acid-6-palmitic acid is particularly preferable.

In the composition for activating autophagy of the present embodiment, fatty acid esters of ascorbyl phosphate or salts thereof may be used alone or in combination of two or more.

When the composition for activating autophagy of the present embodiment contains a fatty acid ester of ascorbyl phosphate or a salt thereof, the amount thereof is preferably 0.05 to 12% by mass, more preferably 0.05 to 5% by mass, and still more preferably 0.1 to 2% by mass.

Fatty acid esters of ascorbyl phosphate or salts thereof can be produced by known production methods such as those described in Japanese Patent No. 62-65550.

For example, as a specific method for producing a fatty acid ester of ascorbyl phosphate, a fatty acid ester of ascorbyl phosphate can be obtained by condensation reaction of the ascorbyl phosphate and a fatty acid or an ester thereof after ascorbyl phosphate is produced in the same manner as the method for producing ascorbyl phosphate described above.

Further, as a specific method for producing a salt of a fatty acid ester of ascorbyl phosphate, a salt of a fatty acid ester of ascorbyl phosphate can be obtained by neutralizing the fatty acid ester solution of ascorbyl phosphate with a metal oxide such as magnesium oxide or a metal hydroxide such as sodium hydroxide.

Examples of commercially available salts of fatty acid esters of ascorbyl phosphate in the composition for activating autophagy of the present embodiment include APPRECIER (registered trademark) (APPS) (compound name: sodium salt of L-ascorbic acid-2-phosphoric acid-6-palmitic acid (also referred to as sodium salt of L-6-O-palmitoyl ascorbic acid-2-phosphoric acid), display name: Trisodium Ascorbyl Palmitate Phosphate) manufactured by Showa Denko K.K.

### <<(iii) Ethyl ascorbic acid of salts thereof>>

### · Ethyl ascorbic acid

Ethyl ascorbic acid is a compound in which an ethyl group is introduced to at least one hydroxyl group of ascorbic acid.

Suitable examples of ethyl ascorbic acid include a compound represented by the following chemical Formula (7).

The compound represented by the following chemical Formula (7) is 3-O-ethyl ascorbic acid in which the hydrogen atom of the hydroxyl group at the third position of ascorbic acid is substituted with an ethyl group.

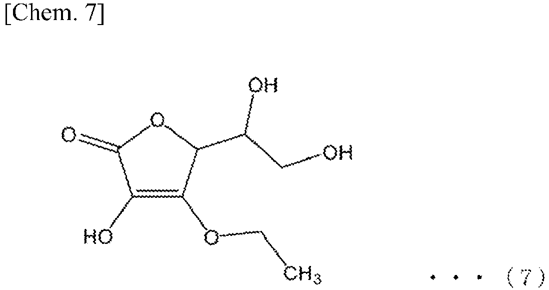

Ethyl ascorbic acid exists as D- and L-stereoisomers, as well as the racemic DL-form. Ethyl ascorbic acid may be any of these stereoisomers, but from the viewpoint of easy availability, the L-form is preferable, and specifically, L-3-O-ethyl ascorbic acid (3-O-ethyl-L-ascorbic acid) is preferable.

### · Salts of ethyl ascorbic acid

Examples of salts of ethyl ascorbic acid include salts of ethyl ascorbic acid and inorganic bases, and salts of ethyl ascorbic acid and organic bases.

Examples of salts with inorganic bases include alkali metal salts such as sodium salts and potassium salts; alkaline earth metal salts such as calcium salts and magnesium salts; aluminum salts; ammonium salts; and zinc salts.

Examples of salts with organic bases include alkylammonium salts and salts with basic amino acids.

Among the above, as ethyl ascorbic acid or salts thereof, from the viewpoint of easy availability, ethyl ascorbic acid is preferable, and L-3-O-ethyl ascorbic acid is more preferable.

In the composition for activating autophagy of the present embodiment, ethyl ascorbic acid or salts thereof may be used alone or in combination of two or more.

When the composition for activating autophagy of the present embodiment contains ethyl ascorbic acid or a salt thereof, the amount thereof is preferably 0.1 to 15% by mass, more preferably 0.5 to 10% by mass, and still more preferably 1 to 5% by mass relative to the total amount of the composition for activating autophagy.

Ethyl ascorbic acid or salts thereof can be produced by a known production method.

For example, as a method for producing ethyl ascorbic acid, ethyl ascorbic acid can be produced by a method of alkylating ascorbic acid with an alkyl halide in the presence of sodium methoxide in dimethyl sulfoxide (DMSO); methods described in Japanese Unexamined Patent Publication No. 8-134055 and Japanese Unexamined Patent Publication No. 1-228977, and the like.

Further, as a specific method for producing a salt of ethyl ascorbic acid, a salt of ethyl ascorbic acid can be obtained by neutralizing the ethyl ascorbic acid solution with a metal oxide such as magnesium oxide or a metal hydroxide such as sodium hydroxide.

Examples of commercially available ethyl ascorbic acid include 3-O-ethyl-L-ascorbic acid (display name: 3-O-ethyl Ascorbic Acid) manufactured by Fujifilm Wako Pure Chemical Industries, Ltd., and the like.

### <<(iv) Ascorbic acid glucoside or salts thereof>>

### · Ascorbic acid glucoside

Ascorbic acid glucoside is a compound in which at least one hydroxyl group of ascorbic acid is glucosidated. The glucosidic bond is preferably an α-glucosidic bond.

Suitable examples of ascorbic acid glucoside include a compound represented by the following chemical Formula (8).

The compound represented by the following chemical Formula (8) is ascorbic acid 2-glucoside in which glucose is bound to the hydroxyl group at the second position of ascorbic acid.

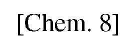

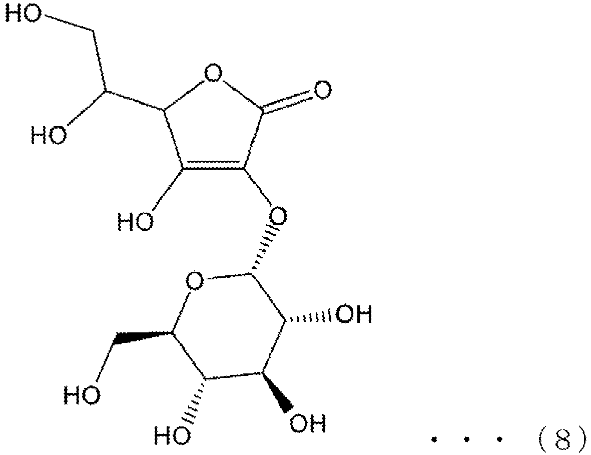

Ascorbic acid exists as D- and L-stereoisomers, as well as the racemic DL-form. Ascorbic acid in ascorbic acid glucoside may be any of these stereoisomers, but from the viewpoint of easy availability, the L-form is preferable, and as ascorbic acid glucoside, L-ascorbic acid 2-glucoside is specifically preferable. Glucose in ascorbic acid glucoside may be in the D-form or the L-form, but the D-form is preferable from the viewpoint of availability.

### · Salts of ascorbic acid glucoside

Examples of salts of ascorbic acid glucoside include salts of ascorbic acid glucoside and inorganic bases, and salts of ascorbic acid glucoside and organic bases.

Examples of salts with inorganic bases include alkali metal salts such as sodium salts and potassium salts; alkaline earth metal salts such as calcium salts and magnesium salts; aluminum salts; ammonium salts; and zinc salts.

Examples of salts with organic bases include alkylammonium salts and salts with basic amino acids.

Among the above, as ascorbic acid glucoside or salts thereof, from the viewpoint of easy availability, ascorbic acid glucoside is preferable, and L-ascorbic acid 2-glucoside is more preferable.

In the composition for activating autophagy of the present embodiment, ascorbic acid glucoside or salts thereof may be used alone or in combination of two or more.

When the composition for activating autophagy of the present embodiment contains ascorbic acid glucoside or a salt thereof, the amount thereof is preferably 0.1 to 15% by mass, more preferably 0.5 to 10% by mass, and still more preferably 1 to 5% by mass relative to the total amount of the composition for activating autophagy.

Ascorbic acid glucoside or salts thereof can be produced, for example, by the method described in Japanese Unexamined Patent Publication No. 03-139288.

For example, as a specific method for producing ascorbic acid glucoside, ascorbic acid glucoside can be produced by α-glucosidic bonding of one molecule of glucose to the hydroxyl group at the second position of ascorbic acid by an enzymatic reaction.

Further, as a specific method for producing a salt of ascorbic acid glucoside, a salt of ascorbic acid glucoside can be obtained by neutralizing the ascorbic acid glucoside solution with a metal oxide such as magnesium oxide or a metal hydroxide such as sodium hydroxide.

Examples of commercially available ascorbic acid glucoside include ascorbic acid 2-glucoside (compound name: L-ascorbic acid 2-glucoside, display name: Ascorbyl Glucoside) manufactured by Hayashibara Co., Ltd., and the like.

In the composition for activating autophagy of the present embodiment, ascorbic acid derivatives or salts thereof may be used alone or in combination of two or more.

As the ascorbic acid derivatives or salts thereof contained in the composition for activating autophagy of the present embodiment, among the above, from the viewpoint of further activating autophagy, (i) ascorbyl phosphate or salts thereof or (ii) fatty acid esters of ascorbyl phosphate or salts thereof are preferable, and (ii) fatty acid esters of ascorbyl phosphate or salts thereof are more preferable.

The composition for activating autophagy of the present embodiment can further promote the expression of the LC3 gene, ATG5 gene, and ATG7 gene by containing an ascorbic acid derivative or a salt thereof in addition to methyl hesperidin.

Moreover, the composition for activating autophagy of the present embodiment can further suppress the expression of mTOR gene by containing an ascorbic acid derivative or a salt thereof in addition to methyl hesperidin.

Further, the composition for activating autophagy of the present embodiment can further promote the expression of the LC3 gene, ATG5 gene, and ATG7 gene in the presence of amyloid β, especially in nerve cells, by containing an ascorbic acid derivative or a salt thereof in addition to methyl hesperidin.

In addition, the composition for activating autophagy of the present embodiment can further suppress apoptosis in the presence of amyloid β, especially in nerve cells, by containing an ascorbic acid derivative or a salt thereof in addition to methyl hesperidin.

<Vitamin E derivative or salt thereof>

The composition for activating autophagy of the present embodiment preferably contains a vitamin E derivative or a salt thereof in addition to the autophagy activator. By containing a vitamin E derivative or a salt thereof, the activation action of autophagy is further improved.

Examples of vitamin E derivatives include tocopherol phosphates or salts thereof.

Examples of tocopherol phosphate include compounds represented by the following general Formula (9).

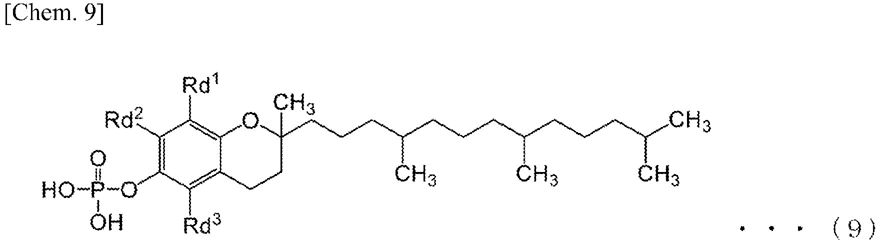

[In the formula, Rd¹, Rd², and Rd³ each independently represent a hydrogen atom or a methyl group.]

In the tocopherol phosphate, according to Rd¹, Rd², and Rd³ in the general Formula (9), α-tocopherol phosphate (Rd¹, Rd², Rd³ = CH₃), β-tocopherol phosphate (Rd¹, Rd³ = CH₃, Rd² = H), γ-tocopherol phosphate (Rd¹, Rd² = CH₃, Rd³ = H), δ-tocopherol phosphate (Rd¹ = CH₃, Rd², Rd³ = H), ζ₂-tocopherol phosphate (Rd², Rd³ = CH₃, Rd¹ = H), η-tocopherol phosphate (Rd² = CH₃, Rd¹, Rd³ = H) and the like are present.

The tocopherol phosphate is not particularly limited and may be any of these tocopherol phosphates. Among these, α-tocopherol phosphate and γ-tocopherol phosphate are preferable, and α-tocopherol phosphate is more preferable.

Since the compound represented by the above general Formula (9) has an asymmetric carbon atom at the second position of the chroman ring, d- and 1-stereoisomers and dl-stereoisomers are present. The tocopherol phosphate may be any of these stereoisomers, but the dl-form is preferable.

Among the above, as the tocopherol phosphate, d1-α-tocopherol phosphate and d1-γ-tocopherol phosphate are preferable, and d1-α-tocopherol phosphate is more preferable.

The salt of tocopherol phosphate is not particularly limited, but examples thereof include salts with inorganic bases and salts with organic bases.

Examples of salts with inorganic bases include alkali metal salts such as sodium salts and potassium salts; alkaline earth metal salts such as calcium salts and magnesium salts; aluminum salts; ammonium salts; and zinc salts.

Examples of salts with organic bases include alkylammonium salts and salts with basic amino acids.

Among the above, as the salt of tocopherol phosphate, an alkali metal salt is preferable, and a sodium salt is more preferable. Alkali metal salts of tocopherol phosphates, particularly sodium salts, have the advantage of being highly soluble in water and easy to handle because alkali metal salts are powdery.

Preferable aspects of the tocopherol phosphate include an alkali metal salt (for example, sodium salt) of the compound represented by the general Formula (9), an alkali metal salt (for example, sodium salt) of α-tocopherol phosphate, an alkali metal salt (for example, sodium salt) of γ-tocopherol phosphate, an alkali metal salt (for example, sodium salt) of d1-α-tocopherol phosphate, and an alkali metal salt (for example, sodium salt) of d1-γ-tocopherol phosphate.

Among the alkali metal salts of tocopherol phosphate, the sodium salt of α-tocopherol phosphate and the sodium salt of γ-tocopherol phosphate are preferable, and the sodium salt of α-tocopherol phosphate is more preferable.

The sodium salt of d1-α-tocopherol phosphate is commercially available from Showa Denko K.K. under the product name of TPNa (registered trademark) (display name: Sodium Tocopheryl Phosphate). The TPNa is exemplary preferable example of the tocopherol phosphate.

In the composition for activating autophagy of the present embodiment, the tocopherol phosphates or salts thereof may be used alone or in combination of two or more.

When the composition for activating autophagy of the present embodiment contains tocopherol phosphate or a salt thereof, the amount thereof is preferably 0.01 to 10% by mass, more preferably 0.05 to 5% by mass, and still more preferably 0.1 to 3% by mass relative to the total amount of the composition for activating autophagy.

A tocopherol phosphate or a salt thereof can be produced by known production methods such as those described in Japanese Unexamined Patent Publication No. 59-44375 and International Publication No. 97/14705.

For example, a tocopherol phosphate can be obtained by allowing a phosphorylating agent such as phosphorus oxychloride to act on tocopherol dissolved in a solvent, followed by appropriate purification after completion of the reaction. Furthermore, a salt of tocopherol phosphate can be obtained by neutralizing the obtained tocopherol phosphate with a metal oxide such as magnesium oxide, a metal hydroxide such as sodium hydroxide, or ammonium hydroxide or alkylammonium hydroxide.

The composition for activating autophagy of the present embodiment can further promote the expression of the LC3 gene, ATG5 gene, and ATG7 gene by containing a tocopherol phosphate or a salt thereof in addition to methyl hesperidin.

Moreover, the composition for activating autophagy of the present embodiment can further suppress the expression of mTOR gene by containing a tocopherol phosphate or a salt thereof in addition to methyl hesperidin.

Further, the composition for activating autophagy of the present embodiment can further promote the expression of the LC3 gene, ATG5 gene, and ATG7 gene in the presence of amyloid β, especially in nerve cells, by containing a tocopherol phosphate or a salt thereof in addition to methyl hesperidin.

In addition, the composition for activating autophagy of the present embodiment can further suppresses apoptosis in the presence of amyloid β, especially in nerve cells, by containing a tocopherol phosphate or a salt thereof in addition to methyl hesperidin.

### <Inositol derivative>

The composition for activating autophagy of the present embodiment preferably contains an inositol derivative in which a saccharide is bound to inositol, in addition to the autophagy activator. By containing an inositol derivative, the activation action of autophagy is further improved. The inositol derivative is a compound composed of inositol and saccharide, and specifically, a compound in which a saccharide is bound to at least one hydroxyl group of inositol.

### · Inositol

Inositol is a cyclic hexahydric alcohol represented by C₆H₆(OH)₆. Inositol includes nine types of stereoisomers such as cis-inositol, epi-inositol, allo-inositol, myo-inositol, muco-inositol, neo-inositol, chiro-inositol (there are D- and L-forms), and scyllo-inositol.

Inositol constituting the inositol derivative is preferably myo-inositol, which has physiological activity, among the above stereoisomers. The structural formula of myo-inositol is shown below.

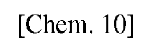

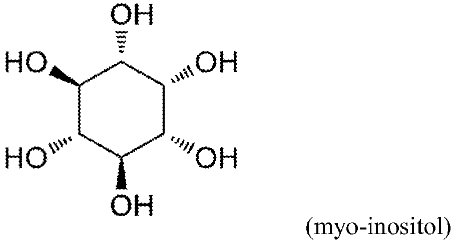

Examples of methods for producing inositol include a method of extracting from rice bran, a chemical synthesis method, and a fermentation method.

### · Saccharide

A saccharide constituting an inositol derivative may be a monosaccharide or an oligosaccharide. Here, monosaccharide means a saccharide that cannot be further hydrolyzed, and means a compound that becomes a component when forming a polysaccharide. A monosaccharide can also be said to be the smallest unit of saccharides. Oligosaccharides are saccharide oligomers in which multiple monosaccharides are bonded by glycosidic bonds.

### ·· Monosaccharide

Specific examples of monosaccharides include glucose (grape sugar), fructose (fruit sugar), galactose, ribose, xylose, mannitol, sorbitol, xylitol, erythritol, and pentaerythritol.

### ··· Oligosaccharide

Specific examples of oligosaccharides include disaccharides such as sucrose (cane sugar), lactose (milk sugar), maltose (malt sugar), isomaltose, trehalose, cellobiose, and maltitol; trisaccharides such as raffinose, melezitose, and maltotriose; tetrasaccharides such as stachyose; hexasaccharides such as α-cyclodextrin; heptasaccharides such as β-cyctodextrin; and octasaccharides such as γ-cyclodextrin.

Among the above, as a saccharide constituting the inositol derivative, glucose or an oligosaccharide containing glucose as a monosaccharide unit is preferable.

Here, a monosaccharide unit means a chemical structure corresponding to a monosaccharide, and can also be said to be a chemical structure derived from a monosaccharide.

The oligosaccharide containing glucose as a monosaccharide unit may be an oligosaccharide in which only glucose is in multiple bonds by glycosidic bonds, or an oligosaccharide in which at least one molecule of glucose and a saccharide other than glucose are in multiple bonds by glycosidic bonds.

The molecular weight of the oligosaccharide containing glucose as a monosaccharide unit may be, for example, approximately 300 to 3000.

In the inositol derivative, the saccharide may be bound to any one of the six hydroxyl groups present in the inositol molecule, or may be bound to any two or more of them. For example, one molecule of inositol may be bound to one or more monosaccharides, one molecule of inositol may be bound to one or more oligosaccharides, and one molecule of inositol may be bound to one or more monosaccharides and one or more oligosaccharides.

In the inositol derivative, the total number of saccharides (monosaccharides and/or oligosaccharides) bound to one molecule of inositol is 1 or more, may be, for example, 2 or more, may be, for example, 3 or more, may be, for example, 4 or more, and may be, for example, 10 or more in terms of monosaccharide units.

Here, conversion to monosaccharide units indicates how many monosaccharide units constitute a saccharide bound to one molecule of inositol. A case where a plurality of saccharides are bound to one molecule of inositol means the sum of number of monosaccharide units of the plurality of saccharides.

Specifically, a disaccharide is converted into 2 monosaccharide units, and a trisaccharide is converted into 3 monosaccharide units. In addition, when a disaccharide and a trisaccharide are bound to one molecule of inositol, the number of monosaccharide units is 5 upon conversion.

More specifically, monosaccharides such as glucose (grape sugar), fructose (fruit sugar), galactose, ribose, xylose, mannitol, sorbitol, xylitol, erythritol, and pentaerythritol converted into a monosaccharide unit.

Disaccharides such as sucrose (cane sugar), lactose (milk sugar), maltose (malt sugar), isomaltose, trehalose, cellobiose, and maltitol are 2 when converted into a monosaccharide unit.

In addition, trisaccharides such as raffinose, melezitose, and maltotriose are 3 when converted into a monosaccharide unit.

In addition, tetrasaccharides such as stachyose are converted into 4 monosaccharide units, hexasaccharides such as α-cyclodextrin are converted into 6 monosaccharide units, heptasaccharides such as β-cyclodextrin are converted into 7 monosaccharide units, and octasaccharides such as γ-cyclodextrin are converted into 8 monosaccharide units.

The inositol derivative preferably uses β-cyclodextrin as a raw material saccharide from the viewpoint of easily obtaining a highly purified inositol derivative. β-cyclodextrin is industrially inexpensive and can be reliably supplied.

In this case, the saccharide constituting the inositol derivative contains glucose as a constituent unit.

On the other hand, when less expensive starch or the like is used as the raw material saccharide for the inositol derivative, various saccharides are transferred to various sites during the synthesis of the inositol derivative, and thus the degree of purification of the obtained inositol derivative tends to be unstable.

Inositol derivatives may be in the form of pharmaceutically acceptable salts.

In the present specification, "pharmaceutically acceptable salt" means a salt form that does not inhibit the physiological activity of the inositol derivative.

The pharmaceutically acceptable salts of inositol derivatives are not particularly limited, and examples thereof include salts with alkali metals (sodium, potassium, and the like); salts with alkaline earth metals (magnesium, calcium, and the like); organic bases (pyridine, triethylamine, and the like), and salts with amines.

Inositol derivatives may also be in the form of solvates. Furthermore, the inositol derivative may be in the form of a solvate of a salt of the inositol derivative. Solvates are not particularly limited, and examples thereof include hydrates and ethanol solvates.

In the composition for activating autophagy of the present embodiment, the inositol derivatives may be used alone or in combination of two or more.

Among the above, the inositol derivative is preferably a mixture of two or more inositol derivatives, more preferably a mixture of 2 to 40 inositol derivatives, still more preferably a mixture of 2 to 30 inositol derivatives, and particularly preferably a mixture of 10 to 30 inositol derivatives.

Among the above, the inositol derivative preferably contains an inositol derivative in which the total number of saccharides bound to one molecule of inositol is 10 or more in terms of monosaccharide units.

In addition, the inositol derivative is preferably an inositol derivative in which glucose or an oligosaccharide containing glucose as a monosaccharide unit is bound to inositol, and a mixture of two or more of the inositol derivatives is preferable. A mixture of 2 to 40 types of inositol derivatives is more preferable, a mixture of 2 to 30 types of inositol derivatives is still more preferable, and a mixture of 10 to 30 types of inositol derivatives is particularly preferable.

Preferably, the inositol derivative contains an inositol derivative in which glucose or an oligosaccharide containing glucose as a monosaccharide unit is bound to inositol, and contains an inositol derivative in which the total of number of glucose molecules bound to one molecule of inositol and oligosaccharides containing glucose as a monosaccharide unit is 10 or more in terms of monosaccharide units.

When the composition for activating autophagy of the present embodiment contains an inositol derivative, the amount thereof is preferably 0.1 to 2% by mass, more preferably 0.2 to 1.5% by mass, and still more preferably 0.5 to 1.5% by mass relative to the total amount of the composition for activating autophagy.

The method for synthesizing the inositol derivative is not particularly limited, and the inositol derivative can be appropriately synthesized by a conventionally known method. For example, inositol and cyclodextrin, which is one type of oligosaccharides, may be reacted in the presence of cyclodextrin glucanotransferase to synthesize an inositol derivative (for example, refer to Japanese Unexamined Patent Publication No. 63-196596). Alternatively, an inositol derivative may be synthesized by a method of obtaining a glucosyl using a glucosyl phosphite as a sugar donor (for example, refer to Japanese Unexamined Patent Publication No. 6-298783).

The composition for activating autophagy of the present embodiment can further promote the expression of the LC3 gene, ATG5 gene, and ATG7 gene by containing an inositol derivative in addition to methyl hesperidin.

Moreover, the composition for activating autophagy of the present embodiment can further suppress the expression of mTOR gene by containing an inositol derivative in addition to methyl hesperidin.

Further, the composition for activating autophagy of the present embodiment can further promote the expression of the LC3 gene, ATG5 gene, and ATG7 gene in the presence of amyloid β, especially in nerve cells, by containing an inositol derivative in addition to methyl hesperidin.

In addition, the composition for activating autophagy of the present embodiment can further suppress apoptosis in the presence of amyloid β, especially in nerve cells, by containing an inositol derivative in addition to methyl hesperidin.

The composition for activating autophagy of the present embodiment may be a pharmaceutical composition or a cosmetic.

### (Pharmaceutical composition)

In one embodiment, the present invention provides a pharmaceutical composition for activating autophagy, containing the autophagy activator described above and a pharmaceutically acceptable carrier.

In the pharmaceutical composition of the present embodiment, the pharmaceutically acceptable carrier is not particularly limited, and in addition to those listed above, carriers generally used for pharmaceuticals can be used. For example, general raw materials described in Japanese Pharmacopoeia, Japanese Non-Pharmacopoeia Pharmaceutical Standards, Pharmaceutical Excipients Standards 2013 (Yakuji Nippo, 2013), Pharmaceutical Excipients Dictionary 2016 (Edited by Japan Pharmaceutical Excipients Association, Yakuji Nippo, 2016), Handbook of Pharmaceutical Excipients, 7th edition (Pharmaceutical Press, 2012), and the like can be used.

The pharmaceutically acceptable carriers may be used alone, or in combination of two or more.

The pharmaceutical composition of the present embodiment may contain other components in addition to the autophagy activator and pharmaceutically acceptable carrier. Other components are not particularly limited, and general pharmaceutical additives can be used. In addition, active components other than the autophagy activators described above can also be used as other components. As the pharmaceutical additives and active components as other components, in addition to the examples above, general raw materials described in Japanese Pharmacopoeia, Japanese Non-Pharmacopoeia Pharmaceutical Standards, Pharmaceutical Excipients Standards 2013 (Yakuji Nippo, 2013), Pharmaceutical Excipients Dictionary 2016 (Edited by Japan Pharmaceutical Excipients Association, Yakuji Nippo, 2016), Handbook of Pharmaceutical Excipients, 7th edition (Pharmaceutical Press, 2012), and the like can be used. The other ingredients may be used alone, or in combination of two or more.

The dosage form of the pharmaceutical composition of the present embodiment is not particularly limited, and may be a dosage form generally used as a pharmaceutical formulation. For example, examples thereof include orally administered dosage forms such as tablets, coated tablets, pills, powders, granules, capsules, liquids, suspensions, and emulsions; and dosage forms for parenteral administration such as injections, suppositories, and external skin preparations. Pharmaceutical compositions in these dosage forms can be formulated according to standard methods (for example, methods described in the Japanese Pharmacopoeia).

The administration method of the pharmaceutical composition of the present embodiment is not particularly limited, and administration can be performed by a method generally used as a pharmaceutical administration method. For example, the pharmaceutical composition may be administered orally as tablets, coated tablets, pills, powders, granules, capsules, liquids, suspensions, and emulsions, may be administered alone as an injection or an infusion preparation or mixed with a general infusion such as glucose solution and Ringer's solution, intravenously, intraarterially, intramuscularly, intradermally, subcutaneously, and intraperitoneally, may be administered rectally as a suppository, and may be administered to the skin as an external skin preparation.

The administration dosage of the pharmaceutical composition of the present embodiment can be a therapeutically effective amount. A therapeutically effective amount may be appropriately determined depending on the symptoms, body weight, age, sex, and the like of the patient, dosage form of the pharmaceutical composition, administration method, and the like.

For example, the administration dosage of the pharmaceutical composition of the present embodiment is 0.01 to 500 mg per dosage unit form as the total amount of methyl hesperidin in the case of oral administration, 0.02 to 250 mg per dosage unit form as the total amount of methyl hesperidin in the case of injection, 0.01 to 500 mg per dosage unit form as the total amount of methyl hesperidin in the case of suppositories, and 0.01 to 500 mg per dosage unit form as the total amount of methyl hesperidin in the case of external skin preparations.

The administration interval of the pharmaceutical composition of the present embodiment may be appropriately determined according to the symptoms, body weight, age, sex, and the like of the patient, dosage form of the pharmaceutical composition, administration method, and the like. For example, administration can be done once a day or approximately 2 to 3 times a day.

The pharmaceutical composition of the present embodiment can be used for treating or preventing diseases caused by decreased autophagy activity. Examples of such diseases include neurodegenerative diseases such as Alzheimer's disease, Huntington's disease, Parkinson's disease, and SENDA disease; inflammatory bowel diseases such as Crohn's disease; and cancer.

The pharmaceutical composition of the present embodiment can be administered to patients with, for example, neurodegenerative diseases such as Alzheimer's disease, Huntington's disease, Parkinson's disease, and SENDA disease; inflammatory bowel diseases such as Crohn's disease; and cancer, and used to suppress progression of neurodegenerative diseases, inflammatory bowel diseases, or cancer. In addition, the pharmaceutical composition of the present embodiment can be administered to patients with neurodegenerative diseases such as Alzheimer's disease, Huntington's disease, Parkinson's disease, and SENDA disease; inflammatory bowel diseases such as Crohn's disease; and cancer, and used to treat neurodegenerative diseases, inflammatory bowel diseases, or cancer. In addition, the pharmaceutical composition of the present embodiment can be used to treat diseases caused by amyloid β. In addition, the pharmaceutical composition of the present embodiment can be used to treat diseases caused by the decreased expression level of the LC3 gene, ATG5 gene, or ATG7 gene. In addition, the pharmaceutical composition of the present embodiment can be used to treat diseases caused by the increased expression level of mTOR.

Among the above, the pharmaceutical composition of the present embodiment can be suitably used for treating Alzheimer's disease.

The pharmaceutical composition of the present embodiment can also be administered to patients at high risk of developing neurodegenerative diseases such as Alzheimer's disease, Huntington's disease, Parkinson's disease, SENDA disease, and the like, and used to prevent neurodegenerative diseases. In addition, the pharmaceutical composition of the present embodiment can also be administered to patients at high risk of developing inflammatory bowel disease such as Crohn's disease, and used to prevent inflammatory bowel diseases. In addition, the pharmaceutical composition of the present embodiment can also be administered to patients at high risk of developing cancer, and used to prevent cancer.

### (Cosmetics)

In one embodiment, the present invention provides a cosmetic for activating autophagy, containing the autophagy activator described above and a pharmaceutically acceptable carrier.

In the cosmetics of the present embodiment, the pharmaceutically acceptable carrier is not particularly limited, and in addition to those listed above, carriers commonly used in cosmetics can be used. For example, general raw materials described in the second edition commentary on the Standards for Cosmetic Ingredients (edited by Society of Japanese Pharmacopoeia, Yakuji Nippo, 1984), Ingredient Standards for Non-Standard Cosmetic Ingredients (supervised by the Examination Division of the Pharmaceutical Affairs Bureau, Ministry of Health and Welfare, Yakuji Nippo, 1993), Supplement to Standards for Non-Standard Cosmetic Ingredients (supervised by the Pharmaceutical Affairs Bureau, Ministry of Health and Welfare, Yakuji Nippo, 1993), Permit criteria for each cosmetic type (supervised by the Pharmaceutical Affairs Bureau, Ministry of Health and Welfare, Yakuji Nippo, 1993), Dictionary of Cosmetic Ingredients (Nikko Chemicals, Inc., 1991), International Cosmetic Ingredient Dictionary and Handbook 2002 Ninth Edition Vol. 1 to 4, by CTFA, and the like can be used.

The pharmaceutically acceptable carriers may be used alone, or in combination of two or more.

The cosmetic of the present embodiment may contain other ingredients in addition to the autophagy activator and pharmaceutically acceptable carrier. Other ingredients are not particularly limited, and general cosmetic additives can be used. In addition, active components other than the autophagy activators described above can also be used as other components. As the cosmetic additives and active components as other components, in addition to the examples above, general raw materials described in the second edition commentary on the Standards for Cosmetic Ingredients (edited by Society of Japanese Pharmacopoeia, Yakuji Nippo, 1984), Ingredient Standards for Non-Standard Cosmetic Ingredients (supervised by the Examination Division of the Pharmaceutical Affairs Bureau, Ministry of Health and Welfare, Yakuji Nippo, 1993), Supplement to Standards for Non-Standard Cosmetic Ingredients (supervised by the Pharmaceutical Affairs Bureau, Ministry of Health and Welfare, Yakuji Nippo, 1993), Permit criteria for each cosmetic type (supervised by the Pharmaceutical Affairs Bureau, Ministry of Health and Welfare, Yakuji Nippo, 1993), Dictionary of Cosmetic Ingredients (Nikko Chemicals, Inc., 1991), International Cosmetic Ingredient Dictionary and Handbook 2002 Ninth Edition Vol. 1 to 4, by CTFA, and the like can be used. The other ingredients may be used alone, or in combination of two or more.

The form of the cosmetic of the present embodiment is not particularly limited, and may be a form commonly used as a cosmetic. Examples thereof include hair cosmetics such as shampoos, rinses, and hair styling agents; basic cosmetics such as facial cleansers, cleansing agents, lotions, milky lotions, lotions, creams, gels, sunscreens, packs, masks, and serums; makeup cosmetics such as foundations, makeup bases, lipsticks, lip glosses, and blushes; and body cosmetics such as body cleansers, body powders, and deodorant cosmetics. These cosmetics can be produced according to standard methods.

In addition, the dosage form of the cosmetic of the present embodiment is not particularly limited, and examples thereof include emulsion type such as oil-in-water (O/W) type, water-in-oil (W/O) type, W/O/W type, and O/W/O type, emulsified polymer type, oily, solid, liquid, dough, stick, volatile oil, powder, jelly, gel, paste, cream, sheet, film, mist, spray, aerosol, multilayer, foam, and flake.

The amount of the cosmetics used in the present embodiment is not particularly limited, but can be an amount effective for activating autophagy.

For example, the amount of the cosmetics used in the present embodiment is 0.01 to 500 mg, may be, for example, 0.15 to 300 mg, may be, for example, 0.15 to 200 mg, and may be, for example 0.2 to 100 mg per use as the total amount of methyl hesperidin.

The use interval of the cosmetic of the present embodiment is not particularly limited, but can be, for example, once a day or approximately 2 to 3 times a day.

The cosmetic of the present embodiment can be used to alleviate symptoms caused by decreased autophagy activity. Alternatively, in order to prevent the development of symptoms caused by decreased autophagy activity, the cosmetic may be used for daily skin care and makeup by test subjects at high risk of developing these symptoms.

### (Other Embodiments)

In one embodiment, the present invention provides a method for activating autophagy, including a step of administering methyl hesperidin to a target.

In one embodiment, the present invention provides a method for promoting expression of the LC3 gene, ATG5 gene, or ATG7 gene, including a step of administering methyl hesperidin to a target.

In one embodiment, the present invention provides a method for suppressing mTOR gene expression, including a step of administering methyl hesperidin to a target.

In one embodiment, the present invention provides a method for suppressing apoptosis in the presence of amyloid β, including a step of administering methyl hesperidin to a target.

In one embodiment, the present invention provides methyl hesperidin for activating autophagy.

In one embodiment, the present invention provides methyl hesperidin for promoting expression of the LC3 gene, ATG5 gene, or ATG7 gene.

In one embodiment, the present invention provides methyl hesperidin for suppressing mTOR gene expression.

In one embodiment, the present invention provides methyl hesperidin for suppressing apoptosis in the presence of amyloid β.

In one embodiment, the present invention provides methyl hesperidin for preventing or treating Alzheimer's disease, Huntington's disease, Parkinson's disease, SENDA disease, Crohn's disease, or cancer.

In one embodiment, the present invention provides the use of methyl hesperidin for producing an autophagy activator.

In one embodiment, the present invention provides the use of methyl hesperidin for producing a promoter of expression of the LC3 gene, ATG5 gene, or ATG7 gene.

In one embodiment, the present invention provides the use of methyl hesperidin for producing a suppressant of mTOR gene expression.

In one embodiment, the present invention provides the use of methyl hesperidin for producing a promoter of expression of the LC3 gene, ATG5 gene, or ATG7 gene in the presence of amyloid β.

In one embodiment, the present invention provides the use of methyl hesperidin for producing a suppressant of apoptosis in the presence of amyloid β.

In one embodiment, the present invention provides the use of methyl hesperidin for producing a composition for activating autophagy.

In one embodiment, the present invention provides the use of methyl hesperidin for producing a composition for promoting expression of the LC3 gene, ATG5 gene, or ATG7 gene.

In one embodiment, the present invention provides the use of methyl hesperidin for producing a composition for suppressing mTOR gene expression.

In one embodiment, the present invention provides the use of methyl hesperidin for producing a composition for promoting expression of the LC3 gene, ATG5 gene, or ATG7 gene in the presence of amyloid β.

In one embodiment, the present invention provides the use of methyl hesperidin for producing a composition for suppressing apoptosis in the presence of amyloid β.

In the above-described embodiments, methyl hesperidin is preferably used in combination with at least one selected from the group consisting of ascorbic acid derivatives or salts thereof, tocopherol phosphates or salts thereof, and inositol derivatives in which a saccharide is bound to inositol.

### [Examples]

The present invention will be described in more detail below with reference to examples, but the present invention is not limited to these examples.

### [Methyl hesperidin]

Methyl hesperidin (product name: Methyl Hesperidin) sold by Showa Denko K.K. was used. In this product, the total amount of the chalcone-1 to chalcone-3 and the flavanone-1 to flavanone-3 is 97.5% by mass or more in the total amount of the composition.

### [Vitamin C derivative]

The following vitamin C derivatives were used in the following examples and prescription examples.
APM: Magnesium salt of L-ascorbic acid-2-phosphate (display name: Magnesium Ascorbyl Phosphate, product name: Ascorbic Acid PM, manufactured by Showa Denko K.K.)
APPS: sodium salt of L-ascorbic acid-2-phosphate-6-palmitic acid (display name: trisodium ascorbyl palmitate phosphate, product name: APPRECIER (APPS), manufactured by Showa Denko K.K.)

### [Vitamin E derivative]

The following vitamin E derivatives were used in the following examples and prescription examples.
α-TPNa: sodium dl-α-tocopheryl phosphate (display name: Sodium Tocopheryl Phosphate, product name: TPNa (registered trademark), manufactured by Showa Denko K.K.)
γ-TPNa: sodium dl-γ-tocopheryl phosphate (manufactured by Showa Denko K.K.)

### [Inositol derivative]

An inositol derivative A produced by the method described in International Publication No. 2019/045113 was used in the following examples and prescription examples.

Specifically, myo-inositol (manufactured by Tsukino Rice Fine Chemicals Co., Ltd.) and β-cyclodextrin (manufactured by Ensuiko Sugar Refining Co., Ltd.) were reacted in the presence of cyclodextrin glucanotransferase (manufactured by Novozymes A/S) to prepare the inositol derivative A, which is a mixture of inositol derivatives in which glucose or an oligosaccharide having glucose as a monosaccharide unit is bound to myo-inositol. As a result of analyzing the prepared inositol derivative A by liquid chromatography-mass spectrometry (LC-MS), the composition was as follows.

**[Table 5]**

| Number of monosaccharide units | Inositol derivative A (% by mass) |
|---|---|
| 1 | 9 |
| 2 | 12 |
| 3 | 12 |
| 4 | 12 |
| 5 | 11 |
| 6 | 9 |
| 7 | 8 |
| 8 | 6 |
| 9 | 5 |
| 10 | 4 |
| 11 | 4 |
| 12 or more | 8 |

### <Preparation of sample solution>

The following sample solutions were prepared and used in Examples and Comparative Examples.
Methyl hesperidin DMSO solution: Methyl hesperidin was dissolved in DMSO.
Hesperidin DMSO solution: Hesperidin (manufactured by Tokyo Chemical Industry Co., Ltd.) was dissolved in DMSO.
APM aqueous solution: APM was dissolved in purified water.
APPS aqueous solution: APPS was dissolved in purified water.
α-TPNa solution: α-TPNa was dissolved in 0.05% (V/V) ethanol aqueous solution.
γ-TPNa solution: γ-TPNa was dissolved in 0.05% (V/V) ethanol aqueous solution.
Inositol derivative A aqueous solution: The inositol derivative A was dissolved in purified water.

### <Evaluation of LC3 gene, ATG5 gene, and ATG7 gene expression promoting effect in human senescent fibroblasts>

To prepare cells that artificially induced senescence, experiments were performed using fibroblasts. Senescent fibroblasts were prepared by the following procedure.

### <<Preparation of senescent fibroblasts>>

Normal human fibroblasts (NB1RGB, RIKEN BRC Cell Bank) were cultured until confluent in D-MEM medium (manufactured by Sigma-Aldrich) to which 10% fetal bovine serum (manufactured by MP Biomedicals) was added. Thereafter, the cells were treated with 250 µM aqueous hydrogen peroxide for 2 hours, and cultured for 24 hours in D-MEM medium to which fresh 10% fetal bovine serum was added. The treatment with aqueous hydrogen peroxide and the cell culture operation were repeated three times, and the obtained fibroblasts were used as senescent fibroblasts.

### «Evaluation test of gene expression promoting effect»

The prepared senescent fibroblasts were prepared at a seeding density of 10000 cells/cm² and cultured for 24 hours in D-MEM medium (manufactured by Sigma-Aldrich) to which 10% fetal bovine serum (manufactured by MP Biomedicals) was added. Next, in Example 1, the methyl hesperidin DMSO solution was added to the medium such that the final concentration of methyl hesperidin was 10⁻³% (V/V) and the final concentration of DMSO was 0.1 % (V/V). In Example 2, the methyl hesperidin DMSO solution was added to the medium such that the final concentration of methyl hesperidin was 10⁻²% (V/V) and the final concentration of DMSO was 0.1% (V/V). In Example 3, the methyl hesperidin DMSO solution was added to the medium such that the final concentration of methyl hesperidin was 10⁻²% (V/V) and the final concentration of DMSO was 0.1% (V/V), and the APM aqueous solution was added to the medium such that the final concentration of APM was 100 µM. In Example 4, the methyl hesperidin DMSO solution was added to the medium such that the final concentration of methyl hesperidin was 10⁻²% (V/V) and the final concentration of DMSO was 0.1% (V/V), and the APPS aqueous solution was added to the medium such that the final concentration of APPS was 10 µM. In Example 5, the methyl hesperidin DMSO solution was added to the medium such that the final concentration of methyl hesperidin was 10⁻²% (V/V) and the final concentration of DMSO was 0.1% (V/V), and the α-TPNa solution was added to the medium such that the final concentration of α-TPNa was 10 µM. In Example 6, the methyl hesperidin DMSO solution was added to the medium such that the final concentration of methyl hesperidin was 10⁻²% (V/V) and the final concentration of DMSO was 0.1% (V/V), and the γ-TPNa solution was added to the medium such that the final concentration of γ-TPNa was 10 µM. In Example 7, the methyl hesperidin DMSO solution was added to the medium such that the final concentration of methyl hesperidin was 10⁻²% (V/V) and the final concentration of DMSO was 0.1% (V/V), and the inositol derivative A aqueous solution was added to the medium such that the final concentration of inositol derivative A was 10⁻³% (V/V).

In addition, in Comparative Example 1, only DMSO was added to the medium such that the final concentration of DMSO was 0.1% (V/V). In Comparative Example 2, the hesperidin DMSO solution was added to the medium such that the final concentration of hesperidin was 10⁻²% (V/V) and the final concentration of DMSO was 0.1% (V/V).

Each medium was then cultured for 24 hours at 37°C and 5% CO₂.

On the other hand, as Reference Example 1, the normal human fibroblasts were prepared at a seeding density of 10000 cells/cm², and cultured for 24 hours in D-MEM medium (manufactured by Sigma-Aldrich) to which 10% fetal bovine serum (manufactured by MP Biomedicals) was added, and only DMSO was added to the medium such that the final concentration of DMSO was 0.1% (V/V). The medium was then cultured for 24 hours at 37°C and 5% CO₂.

Then, using Nucleospin (registered trademark) RNA kit (manufactured by Takara Bio Inc.), RNA was extracted from senescent fibroblasts of normal human fibroblasts in each example, and cDNA was synthesized from the obtained RNA. Next, using this cDNA as a template, by quantitative real-time PCR, the expression level of each gene was quantified using primers (manufactured by Takara Bio Inc.) specific to the LC3 gene, ATG5 gene, and ATG7 gene, respectively.

As an internal standard gene, the expression level of GAPDH (primer manufactured by Takara Bio Inc.), which is a housekeeping gene of which gene expression does not change with the addition of compounds, was quantified, and the expression level of each gene was standardized by the value. Regarding the gene expression level in each of the above examples, the relative gene expression level was obtained when the expression level of each gene in Comparative Example 1 was assumed to be 1.00. The results are shown in Table 6.

**[Table 6]**

| | Autophagy activator or DMSO | Additional ingredients | Cell | Relative gene expression level | | |
|---|---|---|---|---|---|---|
| | | | | LC3 | ATG5 | ATG7 |
| Reference Example 1 | 0.1% (V/V) DMSO | - | Normal fibroblasts | 1396.92 | 1.75 | 1.69 |
| Comparative Example 1 | 0.1% (V/V) DMSO | - | Senescent fibroblasts | 1.00 | 1.00 | 1.00 |
| Comparative Example 2 | 10⁻²% (V/V) hesperidin | - | | < 0 | 0.93 | 0.76 |
| Example 1 | 10⁻³% (V/V) methyl hesperidin | - | | 135.32 | 1.59 | 2.31 |
| Example 2 | 10⁻²% (V/V) methyl hesperidin | - | | 2726.88 | 1.76 | 1.76 |
| Example 3 | 10⁻²% (V/V) methyl hesperidin | 100 µM APM | | 2801.23 | 1.87 | 1.82 |
| Example 4 | 10⁻²% (V/V) methyl hesperidin | 10 µM APPS | | 2586.73 | 1.95 | 1.90 |
| Example 5 | 10⁻²% (V/V) methyl hesperidin | 10 µM α-TPNa | | 2833.19 | 1.97 | 1.91 |
| Example 6 | 10⁻²% (V/V) methyl hesperidin | 10 µM γ-TPNa | | 2811.85 | 1.88 | 1.80 |
| Example 7 | 10⁻²% (V/V) methyl hesperidin | 10⁻³% (V/V) inositol derivative A | | 2798.72 | 1.81 | 1.79 |

As shown in Table 6, in Reference Example 1 and Comparative Example 1, in normal human fibroblasts and senescent fibroblasts cultured with the addition of DMSO only, respectively, it was confirmed that the expression levels of the LC3 gene, ATG5 gene, and ATG7 gene all decreased in the senescent fibroblasts.

When comparing the senescent fibroblasts cultured with the addition of the hesperidin DMSO solution of Comparative Example 2 and the senescent fibroblasts cultured with the addition of DMSO only of Comparative Example 1, in the senescent fibroblasts of Comparative Example 2, the expression levels of the LC3 gene, ATG5 gene, and ATG7 gene more decreased than the senescent fibroblasts of Comparative Example 1.

On the other hand, in senescent fibroblasts cultured with the addition of autophagy activators of Examples 1 to 7, compared to senescent fibroblasts cultured with the addition of DMSO only of Comparative Example 1, the expression levels of the LC3 gene, ATG5 gene, and ATG7 gene were all increased, and in particular, the expression level of the LC3 gene was increased.

In senescent fibroblasts cultured with the addition of methyl hesperidin at a final concentration of 10⁻²% (V/V) of Examples 3 to 7 and additional ingredients (APM, APPS, α-TPNa, γ-TPNa, or inositol derivative A), compared to the senescent fibroblasts cultured with the addition of methyl hesperidin at a final concentration of 10⁻²% (V/V) of Example 2, the expression levels of the LC3 gene, ATG5 gene, and ATG7 gene more increased, and the expression of each of the above genes was further promoted. These results confirmed that the combined use of methyl hesperidin and additional ingredients (APM, APPS, α-TPNa, γ-TPNa, or inositol derivative A) could further promote the expression of the LC3 gene, ATG5 gene, and ATG7 gene.

### <Evaluation of mTOR gene expression suppression effect in human senescent fibroblasts>

The prepared senescent fibroblasts were prepared at a seeding density of 10000 cells/cm² and cultured for 24 hours in D-MEM medium (manufactured by Sigma-Aldrich) to which 10% fetal bovine serum (manufactured by MP Biomedicals) was added. Next, in Example 8, the methyl hesperidin DMSO solution was added to the medium such that the final concentration of methyl hesperidin was 10⁻³% (V/V) and the final concentration of DMSO was 0.1 % (V/V). In Example 9, the methyl hesperidin DMSO solution was added to the medium such that the final concentration of methyl hesperidin was 10⁻²% (V/V) and the final concentration of DMSO was 0.1% (V/V). In Example 10, the methyl hesperidin DMSO solution was added to the medium such that the final concentration of methyl hesperidin was 10⁻²% (V/V) and the final concentration of DMSO was 0.1% (V/V), and the APM aqueous solution was added to the medium such that the final concentration of APM was 100 µM. In Example 11, the methyl hesperidin DMSO solution was added to the medium such that the final concentration of methyl hesperidin was 10⁻²% (V/V) and the final concentration of DMSO was 0.1% (V/V), and the APPS aqueous solution was added to the medium such that the final concentration of APPS was 10 µM. In Example 12, the methyl hesperidin DMSO solution was added to the medium such that the final concentration of methyl hesperidin was 10⁻²% (V/V) and the final concentration of DMSO was 0.1% (V/V), and the α-TPNa solution was added to the medium such that the final concentration of α-TPNa was 10 µM. In Example 13, the methyl hesperidin DMSO solution was added to the medium such that the final concentration of methyl hesperidin was 10⁻²% (V/V) and the final concentration of DMSO was 0.1% (V/V), and the γ-TPNa solution was added to the medium such that the final concentration of γ-TPNa was 10 µM. In Example 14, the methyl hesperidin DMSO solution was added to the medium such that the final concentration of methyl hesperidin was 10⁻²% (V/V) and the final concentration of DMSO was 0.1% (V/V), and the inositol derivative A aqueous solution was added to the medium such that the final concentration of inositol derivative A was 10⁻³% (V/V).

In addition, in Comparative Example 3, DMSO was added to the medium such that the final concentration of DMSO was 0.1% (V/V). In Comparative Example 4, the hesperidin DMSO solution was added to the medium such that the final concentration of hesperidin was 10⁻²% (V/V) and the final concentration of DMSO was 0.1 % (V/V).

Each medium was then cultured for 24 hours at 37°C and 5% CO₂.

On the other hand, as Reference Example 2, the normal human fibroblasts were prepared at a seeding density of 10000 cells/cm², and cultured for 24 hours in D-MEM medium (manufactured by Sigma-Aldrich) to which 10% fetal bovine serum (manufactured by MP Biomedicals) was added, and only DMSO was added to the medium such that the final concentration of DMSO was 0.1% (V/V). The medium was then cultured for 24 hours at 37°C and 5% CO₂.

Then, using Nucleospin (registered trademark) RNA kit (manufactured by Takara Bio Inc.), RNA was extracted from senescent fibroblasts or normal human fibroblasts in each example, and cDNA was synthesized from the obtained RNA. Next, using this cDNA as a template, by quantitative real-time PCR, the expression level of mTOR gene was quantified using primers (manufactured by Takara Bio Inc.) specific to mTOR gene.

As an internal standard gene, the expression level of GAPDH (primer manufactured by Takara Bio Inc.), which is a housekeeping gene of which expression does not change with the addition of compounds, was quantified, and the expression level of each gene was standardized by the value. Regarding the gene expression level in each of the examples, the relative gene expression level was obtained when the expression level of mTOR gene in Comparative Example 3 was assumed to be 1.00. The results are shown in Table 7.

**[Table 7]**

| | Autophagy activator or DMSO | Additional ingredients | Cell | Relative gene expression level |
|---|---|---|---|---|
| | | | | mTOR |
| Reference Example 2 | 0.1% (V/V) DMSO | - | Normal fibroblasts | 0.66 |
| Comparative Example 3 | 0.1% (V/V) DMSO | - | Senescent fibroblasts | 1.00 |
| Comparative Example 4 | 10⁻²% (V/V) hesperidin | - | | 1.16 |
| Example 8 | 10⁻³% (V/V) methyl hesperidin | - | | 0.48 |
| Example 9 | 10⁻²% (V/V) methyl hesperidin | - | | 0.85 |
| Example 10 | 10⁻²% (V/V) methyl hesperidin | 100 µM APM | | 0.78 |
| Example 11 | 10⁻²% (V/V) methyl hesperidin | 10 µM APPS | | 0.66 |
| Example 12 | 10⁻²% (V/V) methyl hesperidin | 10 µM α-TPNa | | 0.65 |
| Example 13 | 10⁻²% (V/V) methyl hesperidin | 10 µM γ-TPNa | | 0.72 |
| Example 14 | 10⁻²% (V/V) methyl hesperidin | 10⁻³% (V/V) inositol derivative A | | 0.73 |

As shown in Table 7, in Reference Example 2 and Comparative Example 3, in normal human fibroblasts and senescent fibroblasts cultured with the addition of DMSO only, respectively, it was observed that mTOR gene expression was promoted in senescent fibroblasts of Comparative Example 3 compared to normal human fibroblasts of Reference Example 2.

The expression level of the mTOR gene in the senescent fibroblasts cultured with the addition of hesperidin DMSO solution of Comparative Example 4 was almost the same as that of the senescent fibroblasts cultured with the addition of DMSO only of Comparative Example 3.

On the other hand, in the senescent fibroblasts cultured with the addition of the autophagy activators of Examples 8 to 14, compared to the senescent fibroblasts cultured with the addition of DMSO only of Comparative Example 3, the expression level of mTOR gene decreased, and the expression of the mTOR gene was suppressed.

In senescent fibroblasts cultured with the addition of methyl hesperidin at a final concentration of 10⁻²% (V/V) of Examples 10 to 14 and additional ingredients (APM, APPS, α-TPNa, γ-TPNa, or inositol derivative A), compared to the senescent fibroblasts cultured with the addition of methyl hesperidin at a final concentration of 10⁻²% (V/V) of Example 9, the expression level of mTOR gene more decreased, and the expression of mTOR gene was further suppressed. These results confirmed that the combined use of methyl hesperidin and additional ingredients (APM, APPS, α-TPNa, γ-TPNa, or inositol derivative A) could further suppress the expression of mTOR gene.

### <Evaluation of LC3 gene, ATG5 gene, and ATG7 gene expression promoting effect in human neuroblastoma>

In the presence of an autophagy activator, the expression levels of the LC3 gene, ATG5 gene, and ATG7 gene in human neuroblastoma (SH-SY5Y; obtained from ATCC) were measured by the following test method, and the effect of promoting the expression of the LC3 gene, ATG5 gene, and ATG7 gene by an autophagy activator was evaluated.

In the following examples and comparative examples, tests were performed by adding amyloid β, which is known to cause a decrease in autophagy in nerve cells, to each medium.

SH-SY5Y cells were prepared at a seeding density of 10000 cells/cm² and cultured for 24 hours in D-MEM medium/Ham's F-12 medium (manufactured by Sigma-Aldrich) to which 10% fetal bovine serum (manufactured by MP Biomedicals) was added. Next, in Example 15, the methyl hesperidin DMSO solution was added to the medium such that the final concentration of methyl hesperidin was 10⁻³% (V/V) and the final concentration of DMSO was 0.1% (V/V). In Example 16, the methyl hesperidin DMSO solution was added to the medium such that the final concentration of methyl hesperidin was 10⁻²% (V/V) and the final concentration of DMSO was 0.1 % (V/V). In Example 17, the methyl hesperidin DMSO solution was added to the medium such that the final concentration of methyl hesperidin was 10⁻²% (V/V) and the final concentration of DMSO was 0.1% (V/V), and the APM aqueous solution was added to the medium such that the final concentration of APM was 100 µM. In Example 18, the methyl hesperidin DMSO solution was added to the medium such that the final concentration of methyl hesperidin was 10⁻²% (V/V) and the final concentration of DMSO was 0.1% (V/V), and the APPS aqueous solution was added to the medium such that the final concentration of APPS was 10 µM. In Example 19, the methyl hesperidin DMSO solution was added to the medium such that the final concentration of methyl hesperidin was 10⁻²% (V/V) and the final concentration of DMSO was 0.1% (V/V), and the α-TPNa solution was added to the medium such that the final concentration of α-TPNa was 10 µM. In Example 20, the methyl hesperidin DMSO solution was added to the medium such that the final concentration of methyl hesperidin was 10⁻²% (V/V) and the final concentration of DMSO was 0.1% (V/V), and the γ-TPNa solution was added to the medium such that the final concentration of γ-TPNa was 10 µM. In Example 21, the methyl hesperidin DMSO solution was added to the medium such that the final concentration of methyl hesperidin was 10⁻²% (V/V) and the final concentration of DMSO was 0.1% (V/V), and the inositol derivative A aqueous solution was added to the medium such that the final concentration of inositol derivative A was 10⁻³% (V/V).

In addition, in Comparative Example 5, DMSO was added to the medium such that the final concentration of DMSO was 0.1% (V/V). In Comparative Example 6, the hesperidin DMSO solution was added to the medium such that the final concentration of hesperidin was 10⁻²% (V/V) and the final concentration of DMSO was 0.1% (V/V).

Next, an amyloid β solution was prepared by dissolving amyloid β (manufactured by Sigma-Aldrich) in a 0.01% (V/V) DMSO aqueous solution, and the amyloid β solution was added to each medium such that the final concentration of amyloid β in each medium was 20 µM.

In addition, in Reference Example 3, only DMSO was added and no amyloid β solution was added such that the final concentration of DMSO was 0.1% (V/V).

Each medium was then cultured for 48 hours at 37°C and 5% CO₂.

Then, using Nucleospin (registered trademark) RNA kit (manufactured by Takara Bio Inc.), RNA was extracted from SH-SY5Y cells in each example, and cDNA was synthesized from the obtained RNA. Next, using this cDNA as a template, by quantitative real-time PCR, the expression level of each gene was quantified using primers (manufactured by Takara Bio Inc.) specific to the LC3 gene, ATG5 gene, and ATG7 gene, respectively.

As an internal standard gene, the expression level of GAPDH (primer manufactured by Takara Bio Inc.), which is a housekeeping gene of which expression does not change with the addition of compounds, was quantified, and the expression level of each gene was standardized by the value. Regarding the gene expression level in each of the examples, the relative gene expression level was obtained when the expression level of each gene in Reference Example 3 was assumed to be 1.00. The results are shown in Table 8.

**[Table 8]**

| | Autophagy activator or DMSO | Additional ingredients | Amyloid β | Relative gene expression level | | |
|---|---|---|---|---|---|---|
| | | | | LC3 | ATG5 | ATG7 |
| Reference Example 3 | 0.1% (V/V) DMSO | - | Absent | 1.00 | 1.00 | 1.00 |
| Comparative Example 5 | 0.1% (V/V) DMSO | - | Present | 0.56 | 0.88 | 0.63 |
| Comparative Example 6 | 10⁻²% (V/V) hesperidin | - | | 2.41 | 3.29 | 2.31 |
| Example 15 | 10⁻³% (V/V) methyl hesperidin | - | | 3.32 | 2.24 | 2.77 |
| Example 16 | 10⁻²% (V/V) methyl hesperidin | - | | 6.52 | 4.97 | 3.97 |
| Example 17 | 10⁻²% (V/V) methyl hesperidin | 100 µM APM | | 6.99 | 5.43 | 4.44 |
| Example 18 | 10⁻²% (V/V) methyl hesperidin | 10 µM APPS | | 7.27 | 5.89 | 5.03 |
| Example 19 | 10⁻²% (V/V) methyl hesperidin | 10 µM α-TPNa | | 7.66 | 6.01 | 5.22 |
| Example 20 | 10⁻²% (V/V) methyl hesperidin | 10 µM γ-TPNa | | 7.32 | 5.54 | 4.65 |
| Example 21 | 10⁻²% (V/V) methyl hesperidin | 10⁻³% (V/V) inositol derivative A | | 6.95 | 5.36 | 4.22 |

As shown in Table 8, in Reference Example 3 and Comparative Example 5, compared to SH-SY5Y cells cultured with the addition of DMSO only, in SH-SY5Y cells cultured with the addition of amyloid β, it was confirmed that the expression levels of the LC3 gene, ATG5 gene, and ATG7 gene all further decreased.

In the SH-SY5Y cells cultured with the addition of the autophagy activator and the amyloid β solution of Examples 15 to 21, compared to the SH-SY5Y cells cultured with the addition of DMSO and the amyloid β solution of Comparative Example 5, the expression levels of the LC3 gene, ATG5 gene, and ATG7 gene all increased.

Moreover, even in the SH-SY5Y cells cultured with the addition of hesperidin at a final concentration of 10⁻²% (V/V) of Comparative Example 6, compared to the SH-SY5Y cells of Comparative Example 5, the expression levels of the LC3 gene, ATG5 gene, and ATG7 gene increased. However, in the SH-SY5Y cells cultured with the addition of methyl hesperidin at a final concentration of 10⁻²% (V/V) of Example 16, the increase in the expression level was remarkable in all genes.

In SH-SY5Y cells cultured with the addition of methyl hesperidin at a final concentration of 10⁻²% (V/V) of Examples 17 to 21 and additional ingredients (APM, APPS, α-TPNa, γ-TPNa, or inositol derivative A), compared to the SH-SY5Y cells cultured with the addition of methyl hesperidin at a final concentration of 10⁻²% (V/V) of Example 16, the expression levels of the LC3 gene, ATG5 gene, and ATG7 gene more increased, and the expression of each of the above genes was further promoted. These results confirmed that the combined use of methyl hesperidin and additional ingredients (APM, APPS, α-TPNa, γ-TPNa, or inositol derivative A) could further promote the expression of the LC3 gene, ATG5 gene, and ATG7 gene in the nerve cells in the presence of amyloid β.

<Evaluation of apoptosis suppressing action caused by amyloid β in human neuroblastoma>

The proportion of apoptotic cells in human neuroblastoma (SH-SY5Y; obtained from ATCC) in the presence of an autophagy activator was measured by the following test method to evaluate the apoptosis suppressing action of the autophagy activator.

In the following examples and comparative examples, tests were performed by adding amyloid β, which is known to induce cell death called apoptosis of nerve cells due to a decrease in autophagy, to each medium.

SH-SY5Y cells were prepared at a seeding density of 50000 cells/cm² and cultured for 24 hours in D-MEM medium/Ham's F-12 medium (manufactured by Sigma-Aldrich) to which 10% fetal bovine serum (manufactured by MP Biomedicals) was added. Next, in Example 22, the methyl hesperidin DMSO solution was added to the medium such that the final concentration of methyl hesperidin was 10⁻³% (V/V) and the final concentration of DMSO was 0.1% (V/V). In Example 23, the methyl hesperidin DMSO solution was added to the medium such that the final concentration of methyl hesperidin was 10⁻²% (V/V) and the final concentration of DMSO was 0.1% (V/V). In Example 24, the methyl hesperidin DMSO solution was added to the medium such that the final concentration of methyl hesperidin was 10⁻²% (V/V) and the final concentration of DMSO was 0.1% (V/V), and the APM aqueous solution was added to the medium such that the final concentration of APM was 100 µM. In Example 25, the methyl hesperidin DMSO solution was added to the medium such that the final concentration of methyl hesperidin was 10⁻²% (V/V) and the final concentration of DMSO was 0.1% (V/V), and the APPS aqueous solution was added to the medium such that the final concentration of APPS was 10 µM. In Example 26, the methyl hesperidin DMSO solution was added to the medium such that the final concentration of methyl hesperidin was 10⁻²% (V/V) and the final concentration of DMSO was 0.1% (V/V), and the α-TPNa solution was added to the medium such that the final concentration of α-TPNa was 10 µM. In Example 27, the methyl hesperidin DMSO solution was added to the medium such that the final concentration of methyl hesperidin was 10⁻²% (V/V) and the final concentration of DMSO was 0.1 % (V/V), and the γ-TPNa solution was added to the medium such that the final concentration of γ-TPNa was 10 µM. In Example 28, the methyl hesperidin DMSO solution was added to the medium such that the final concentration of methyl hesperidin was 10⁻²% (V/V) and the final concentration of DMSO was 0.1% (V/V), and the inositol derivative A aqueous solution was added to the medium such that the final concentration of inositol derivative A was 10⁻³% (V/V).

In addition, in Comparative Example 7, DMSO was added to the medium such that the final concentration of DMSO was 0.1% (V/V). In Comparative Example 8, the hesperidin DMSO solution was added to the medium such that the final concentration of hesperidin was 10⁻²% (V/V) and the final concentration of DMSO was 0.1% (V/V).

Next, an amyloid β solution was prepared by dissolving amyloid β (manufactured by Sigma-Aldrich) in a 0.01% (V/V) DMSO aqueous solution, and the amyloid β solution was added to each medium such that the final concentration of amyloid β in each medium was 30 µM.

In addition, in Reference Example 4, only DMSO was added and no amyloid β solution was added such that the final concentration of DMSO was 0.1% (V/V).

Each medium was then cultured for 48 hours at 37°C and 5% CO₂.

Thereafter, an aqueous solution of 10 µM Hoechst 33342 (manufactured by Sigma-Aldrich) was added to each SH-SY5Y cell from which the medium was removed, and the cells were allowed to stand at room temperature (25°C) for 10 minutes. After removing the solution, each SH-SY5Y cell was washed with phosphate buffer (PBS, manufactured by Wako Pure Chemical Industries, Ltd.), and with a fluorescence microscope, the number of cells showing strong apoptosis-like Hoechst fluorescence due to chromatin aggregation (Hoechst (+) cells) was counted. 5000 or more cells were counted in each visual field in four independent tests, and the average value of the proportion of Hoechst (+) cells in the four tests was taken as the proportion of cells undergoing apoptosis. For each example, relative values were calculated when the proportion of apoptotic cells in Reference Example 4 was set to 1.00, and the results are shown in Table 9.

**[Table 9]**

| | Autophagy activator or DMSO | Additional ingredients | Amyloid β | Proportion of apoptotic cells LC3 |
|---|---|---|---|---|
| Reference Example 4 | 0.1% (V/V) DMSO | - | Absent | 1.00 |
| Comparative Example 7 | 0.1% (V/V) DMSO | - | Present | 7.38 |
| Comparative Example 8 | 10⁻²% (V/V) hesperidin | - | | 3.76 |
| Example 22 | 10⁻³% (V/V) methyl hesperidin | - | | 4.02 |
| Example 23 | 10⁻²% (V/V) methyl hesperidin | - | | 2.17 |
| Example 24 | 10⁻²% (V/V) methyl hesperidin | 100 µM APM | | 1.46 |
| Example 25 | 10⁻²% (V/V) methyl hesperidin | 10 µM APPS | | 1.31 |
| Example 26 | 10⁻²% (V/V) methyl hesperidin | 10 µM α-TPNa | | 1.20 |
| Example 27 | 10⁻²% (V/V) methyl hesperidin | 10 µM γ-TPNa | | 1.22 |
| Example 28 | 10⁻²% (V/V) methyl hesperidin | 10⁻³% (V/V) inositol derivative A | | 1.11 |

As shown in Table 9, in Reference Example 4 and Comparative Example 7, compared to SH-SY5Y cells cultured with the addition of DMSO only of Reference Example 4, in the SH-SY5Y cells cultured with further addition of amyloid β of Comparative Example 7, it was confirmed that the proportion of apoptotic cells increased.

In the SH-SY5Y cells cultured with the addition of the autophagy activator and the amyloid β solution of Examples 22 to 28, compared to the SH-SY5Y cells cultured with the addition of DMSO and the amyloid β solution of Comparative Example 7, it was confirmed that the proportion of apoptotic cells decreased.

Moreover, even in the SH-SY5Y cells cultured with the addition of hesperidin at a final concentration of 10⁻²% (V/V) of Comparative Example 8, compared to the SH-SY5Y cells in Comparative Example 7, the proportion of apoptotic cells decreased. However, in the SH-SY5Y cells cultured with the addition of methyl hesperidin at a final concentration of 10⁻²% (V/V) of Example 23, the decrease in the proportion of apoptotic cells was remarkable.

In SH-SY5Y cells cultured with the addition of methyl hesperidin at a final concentration of 10⁻²% (V/V) of Examples 24 to 28 and additional ingredients (APM, APPS, α-TPNa, γ-TPNa, or inositol derivative A), compared to the SH-SY5Y cells cultured with the addition of methyl hesperidin at a final concentration of 10⁻²% (V/V) of Example 23, the proportion of apoptotic cells more decreased. These results confirmed that the combined use of methyl hesperidin and additional ingredients (APM, APPS, α-TPNa, γ-TPNa, or inositol derivative A) could further suppress the apoptosis of nerve cells in the presence of amyloid β.

### [Prescription Examples]

Table 10 shows Prescription Examples 1 to 5 of external preparations as compositions for activating autophagy.

**[Table 10]**

| Material | Prescription Example 1 (% by mass) | Prescription Example 2 (% by mass) | Prescription Example 3 (% by mass) | Prescription Example 4 (% by mass) | Prescription Example 5 (% by mass) |
|---|---|---|---|---|---|
| Methyl hesperidin | 0.20 | 0.20 | 0.20 | 0.20 | 0.20 |
| Butyl glycol | 3.00 | 3.00 | 3.00 | 3.00 | 3.00 |
| Dipropylene glycol | 2.00 | 2.00 | 2.00 | 2.00 | 2.00 |
| Glvcerin | 3.00 | 3.00 | 3.00 | 3.00 | 3.00 |
| Pentylene glycol | 1.50 | 1.50 | 1.50 | 1.50 | 1.50 |
| Phenoxyethanol | 0.35 | 0.35 | 0.35 | 0.35 | 0.35 |
| Bis-ethoxydiglycol cyclohexane 1,4-dicarboxylate | 0.20 | 0.20 | 0.20 | 0.20 | 0.20 |
| APM | - | 3.00 | - | - | 3.00 |
| APPS | - | - | 0.50 | - | - |
| α-TPNa | - | - | - | 2.00 | - |
| Inositol derivative A | - | - | - | - | 1.00 |
| Water | 89.75 | 86.95 | 89.25 | 87.75 | 88.75 |
| Sum | 100.0 | 100.0 | 100.0 | 100.0 | 100.0 |

### [Industrial Applicability]

According to the present invention, an autophagy activator and a composition for activating autophagy containing the autophagy activator capable of effectively activating autophagy are provided.

Although preferable examples of the present invention have been described above, the present invention is not limited to these examples. Configuration additions, omissions, substitutions, and other changes are possible without departing from the scope of the present invention. The present invention is not limited by the description above, but only by the scope of the appended claims.

## Claims

1. An autophagy activator comprising methyl hesperidin as an active ingredient.

2. The autophagy activator according to claim 1, wherein
the methyl hesperidin is one or more selected from the group consisting of chalcone methyl hesperidin represented by the following general Formula (1) and flavanone methyl hesperidin represented by the following general Formula (2), 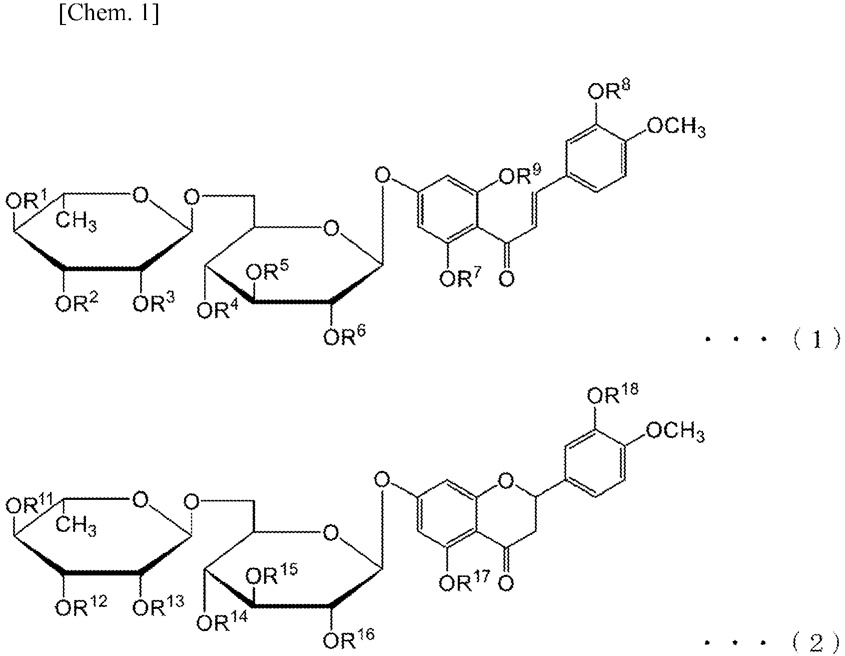
wherein in Formula (1), R¹ to R⁹ are each independently a methyl group or a hydrogen atom, provided that at least one of R¹ to R⁹ is a methyl group; and
in Formula (2), R¹¹ to R¹⁸ are each independently a methyl group or a hydrogen atom, provided that at least one of R¹¹ to R¹⁸ is a methyl group.

3. The autophagy activator according to claim 2, wherein
the methyl hesperidin is one or more selected from the group consisting of chalcone methyl hesperidin represented by the following general Formula (3) and flavanone methyl hesperidin represented by the following general Formula (4), 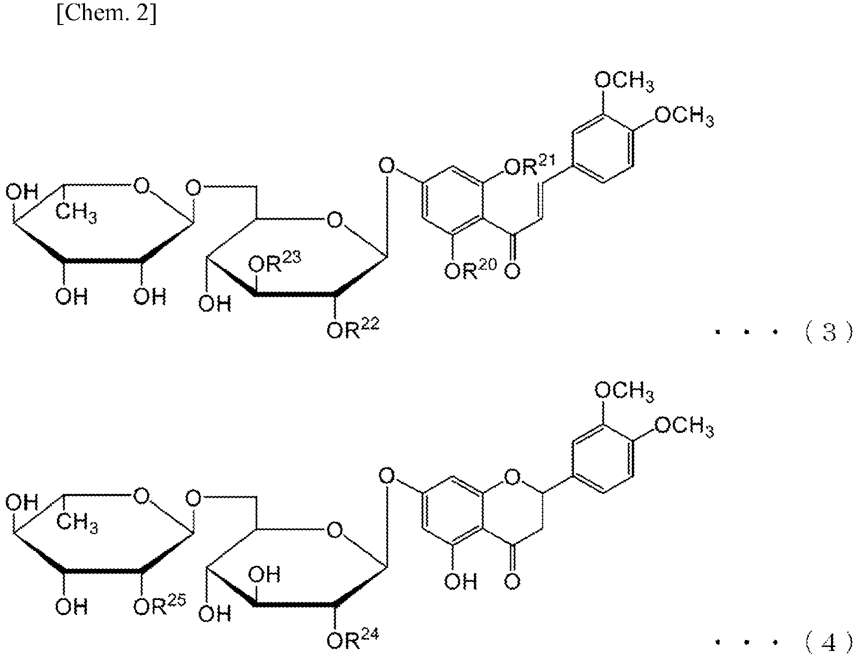
wherein in Formula (3), R²⁰ to R²³ are each independently a methyl group or a hydrogen atom, and
in Formula (4), R²⁴ and R²⁵ are each independently a methyl group or a hydrogen atom.

4. The autophagy activator according to claim 3, wherein
the chalcone methyl hesperidin represented by the general Formula (3) is one or more selected from the group consisting of chalcone-1 to chalcone-3 having a combination of R²⁰ to R²³ shown in Table 1 below.
**[Table 1]**
| | R²⁰ | R²¹ | R²² | R²³ |
|---|---|---|---|---|
| Chalcone-1 | CH₃ | CH₃ | CH₃ | CH₃ |
| Chalcone-2 | H | CH₃ | CH₃ | H |
| Chalcone-3 | H | CH₃ | H | H |

5. The autophagy activator according to claim 3 or 4, wherein
the flavanone methyl hesperidin represented by the general Formula (4) is one or more selected from the group consisting of flavanone-1 to flavanone-4 having a combination of R²⁴ and R²⁵ shown in Table 2 below.
**[Table 2]**
| | R²⁴ | R²⁵ |
|---|---|---|
| Flavanone-1 | CH₃ | CH₃ |
| Flavanone-2 | CH₃ | H |
| Flavanone-3 | H | H |
| Flavanone-4 | H | CH₃ |

6. The autophagy activator according to any one of claims 1 to 5, wherein
LC3 gene expression is promoted.

7. The autophagy activator according to any one of claims 1 to 6, wherein
ATG5 gene expression is promoted.

8. The autophagy activator according to any one of claims 1 to 7, wherein
ATG7 gene expression is promoted.

9. The autophagy activator according to any one of claims 1 to 8, wherein mTOR gene expression is suppressed.

10. The autophagy activator according to any one of claims 1 to 9, which is used for prevention or treatment of Alzheimer's disease.

11. A composition for activating autophagy, comprising the autophagy activator according to any one of claims 1 to 10 and a pharmaceutically acceptable carrier.

12. The composition for activating autophagy according to claim 11, wherein
a total amount of methyl hesperidin is 0.01 to 2% by mass relative to a total amount of the composition for activating autophagy.

13. The composition for activating autophagy according to claim 11 or 12, further comprising at least one vitamin derivative or a salt thereof selected from the group consisting of vitamin C derivatives and vitamin E derivatives.

14. The composition for activating autophagy according to claim 13, wherein
the vitamin derivative or a salt thereof is at least one selected from the group consisting of ascorbyl phosphate, fatty acid esters of ascorbyl phosphate, tocopherol phosphate, and salts thereof.

15. The composition for activating autophagy according to any one of claims 11 to 14, further comprising an inositol derivative in which a saccharide is bound to inositol.

16. The composition for activating autophagy according to claim 15, wherein
the saccharide is glucose or an oligosaccharide containing glucose as a constituent unit.
